# FASCICULE DE BREVET EUROPEEN

(11) **EP 3 876 948 B1**
(45) Date de publication et mention de la délivrance du brevet: **16.11.2022**
(21) Numéro de dépôt: 19812682.3
(22) Date de dépôt: 08.11.2019
(51) Int. Cl.: A61K 31/713, A61P 1/12, A61P 1/04

(54) **UTILISATION DU MICRORNA MIR-27A-5P POUR TRAITER L'INFLAMMATION INTESTINALE INDUITE PAR CLOSTRIDIUM DIFFICILE**
VERWENDUNG DER MIR-27A-5P MIKRORNA ZUR BEHANDLUNG VON CLOSTRIDIUM-DIFFICILE-INDUZIERTER DARMENTZÜNDUNG
USE OF THE MIR-27A-5P MICRORNA FOR TREATING CLOSTRIDIUM DIFFICILE-INDUCED BOWEL INFLAMMATION

(30) Priorité: 09.11.2018 FR 1860380
(43) Date de publication de la demande: 15.09.2021
(73) Titulaire: Université Paris-Saclay, 91190 Gif-sur-Yvette (FR); Assistance Publique - Hôpitaux de Paris, 75004 Paris (FR)
(72) Inventeur: KANSAU, Imad, 75016 PARIS (FR); KOBEISSY, Philippe Hussein, 92160 ANTONY (FR); LARRAZET, Cécile, 78180 MONTIGNY-LE BRETONNEUX (FR); MARVAUD, Jean-Christophe, 78000 VERSAILLES (FR)
(74) Mandataire: Regimbeau
(86) Numéro de dépôt international: PCT/EP2019/080748
(87) Numéro de publication internationale: WO 2020/094865

(56) Documents cités:
- EP-A2- 2 021 499
- KR-A- 20150 043 789
- Anonymous: "Immunoregulatory mechanisms of microRNAs during Clostridium difficile infection | Nutritional Immunology and Molecular Medicine Laboratory", , 1 janvier 2014 (2014-01-01), XP055589249, Extrait de l'Internet: URL:https://www.nimml.org/publications/pos ter/immunoregulatory-mechanisms-of-microrn as-during-clostridium-difficile-infec [extrait le 2019-05-16]
- Anonymous: "Archive History for NCT01930032", , 6 mars 2017 (2017-03-06), XP055589255, Extrait de l'Internet: URL:https://clinicaltrials.gov/ct2/history /NCT01930032?V_6=View#StudyPageTop [extrait le 2019-05-16]
- KURT FISHER ET AL: "MicroRNA in inflammatory bowel disease: Translational research and clinical implication", WORLD JOURNAL OF GASTROENTEROLOGY, vol. 21, no. 43, 1 janvier 2015 (2015-01-01), pages 12274-12282, XP055589256, CN ISSN: 1007-9327, DOI: 10.3748/wjg.v21.i43.12274
- YING CHENG ET AL: "Mmu-miR-27a-5p-Dependent Upregulation of MCPIP1 Inhibits the Inflammatory Response in LPS-Induced RAW264.7 Macrophage Cells", BIOMED RESEARCH INTERNATIONAL, vol. 2015, 1 janvier 2015 (2015-01-01), pages 1-10, XP055589271, ISSN: 2314-6133, DOI: 10.1155/2015/607692

## Description

### Résumé de l'invention

Les présents inventeurs ont identifié un miRNA capable de fortement moduler la réponse inflammatoire induite par *Clostridium difficile.* Ils proposent donc d'incorporer ce miRNA dans une composition pharmaceutique pour prévenir ou diminuer les effets délétères de cette infection sur les intestins des patients infectés. Ce microRNA est le miR-27a-5p. De manière avantageuse, ce miRNA peut être associé à une matrice, incorporé dans des particules, ou véhiculé par un vecteur. Il peut être administré à des sujets infectés à des fins thérapeutiques.

### Description de l'art antérieur

*Clostridium difficile* est la principale bactérie entéropathogène responsable d'infections post-antibiotiques dans les pays industrialisés [1, 2]. L'exposition aux antibiotiques, l'hospitalisation et l'âge avancé sont les principaux facteurs de risque associés à l'infection à *C. difficile* (ICD) [1]. L'ICD peut aller d'une diarrhée légère à une colite pseudomembraneuse grave menaçant le pronostic vital.

L'augmentation de l'incidence et de la sévérité des ICDs ces dernières années coïncide avec l'émergence de clones épidémiques, en particulier des souches de ribotype 027. Cette augmentation a été accompagnée d'une augmentation des coûts des soins [3, 4]. Selon les estimations, le coût des traitements associés aux ICDs aux Etats-Unis s'élève à 3 milliards de dollars par an avec des projections similaires pour le Canada [5-8]. Aux États-Unis, le fardeau économique annuel des ICDs sur le système de santé est estimé à près de 4,8 milliards de dollars par an en coûts excédentaires dans les établissements de soins aigus [3]. La plupart de ces coûts ont été attribués à des épisodes primaires d'ICD, avec un coût de 12 607 $ par cas [9]. En Europe, les coûts de prise en charge des ICD estimés par le Centre européen de prévention et contrôle des maladies (CEPCM) atteignent près de 4,4 milliards de dollars par an [10]. En douze ans, les surcoûts des ICDs peuvent avoir augmenté de 27 à 93 % selon les pays [11 ; 39]. En France, les estimations de surcoûts peuvent varier de 1 666 à 5 867 € par cas d'ICD [12].

Ces coûts élevés sont en grande partie liés à la nécessité d'isolement des patients et à la prolongation des durées de séjours hospitaliers. Cependant, la charge totale de I'ICD est susceptible d'être largement sous-estimée, puisque les coûts de récurrences, les événements indésirables causés par I'ICD (complications), le coût des soins dans les établissements de soins de longue durée et les coûts sociaux doivent encore être définis. C'est le cas également pour les ICD communautaires (hors hôpital).

Les toxines cytolytiques de *C. difficile* A et B (respectivement connues sous le nom de TcdA et TcdB) contribuent directement à la pathologie associée aux ICD [13, 14], mais d'autres facteurs jouent un rôle dans la colonisation et la pathogénicité de la bactérie. Parmi ces facteurs, les flagelles conférant la mobilité et le chimiotactisme à la bactérie [15], joueraient un rôle dans la réponse inflammatoire intestinale par l'activation des voies de signalisation des MAPKs (Mitogen-activated Protein Kinases) et de NF-κB, via l'interaction avec le récepteur cellulaire de la réponse immunitaire innée TLR5 (pour Toll-like receptor 5) [16]. Dans cette réponse pro-inflammatoire, la voie de NF-κB semble être la voie prédominante. Par ailleurs, *in vivo* dans un modèle murin d'ICD, les toxines agiraient en coopération avec les flagelles de C. *difficile* pour induire une réponse inflammatoire, suggérant le rôle synergique des toxines et des flagelles dans la réponse inflammatoire intestinale [17]. La sévérité de l'inflammation intestinale est corrélée à la gravité des présentations cliniques des ICD.

Différents mécanismes de régulation des voies de signalisation des récepteurs cellulaires de la réponse immunitaire innée, les TLRs *(Toll-Like Receptors),* ont été décrits. Parmi ces mécanismes, les microRNA (miRNA), des séquences d'ARN non-codant de 21 à 24 nucléotides, responsables de la régulation post-transcriptionnelle de gènes par leur action spécifique sur les ARNm [18], jouent un rôle d'immunomodulateurs de la réponse innée par la régulation des voies dépendantes des TLRs. Les miRNAs, dont l'expression serait induite par l'activation des TLRs, modulent la réponse inflammatoire par l'inhibition d'effecteurs des cascades de signalisation. Ils agiraient sur l'expression des cibles effectrices, des facteurs de transcription des voies de l'inflammation et des récepteurs TLR eux-mêmes [19, 20].

A ce jour, il n'a jamais été démontré ni suggéré qu'il était possible de diminuer efficacement la réponse inflammatoire intestinale induite par *Clostridium difficile,* en utilisant un miRNA modulant la voie de signalisation des TLR, par exemple la voie NF-KB.

Or les miRNAs sont des molécules connues pour leur absence d'effets délétères sur les tissus normaux. Ils sont aujourd'hui considérés comme des candidats médicament efficaces de nouvelle génération, notamment pour traiter les cancers ([40]).

Dans ce contexte, il serait très avantageux d'identifier un miRNA capable de fortement moduler la réponse inflammatoire induite par *Clostridium difficile,* par exemple en inhibant de façon suffisamment forte la voie NF-κB suractivée lors de cette infection. L'incorporation d'un tel miRNA dans une composition pharmaceutique permettrait d'obtenir un médicament capable de prévenir ou diminuer les effets délétères de cette bactérie sur les intestins des patients infectés.

### Description détaillée de l'invention

Dans cette logique, les présents inventeurs ont étudié l'expression d'un panel de miRNAs potentiellement impliqués dans la voie de signalisation TLR5-NF-κB *in vitro* et *in vivo,* dans le modèle de cellules épithéliales intestinales humaines Caco-2 et dans un modèle murin d'ICD.

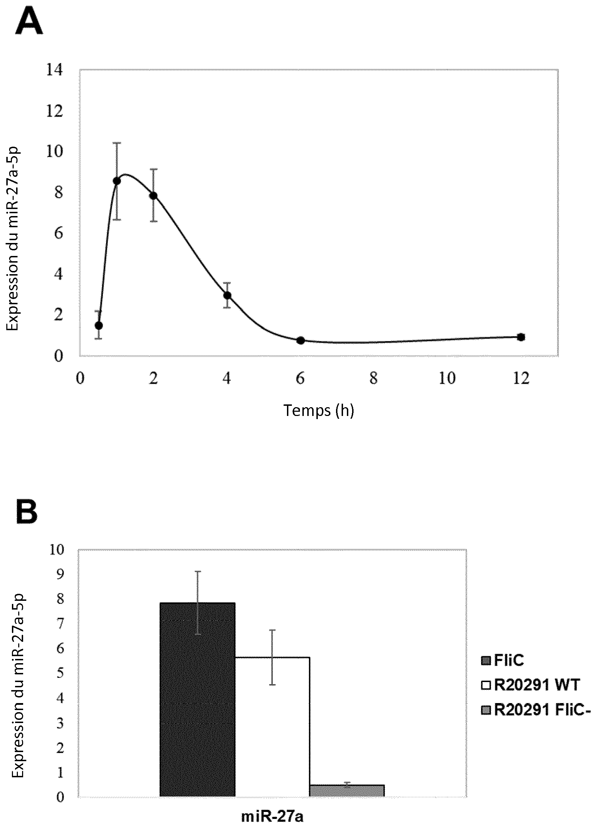

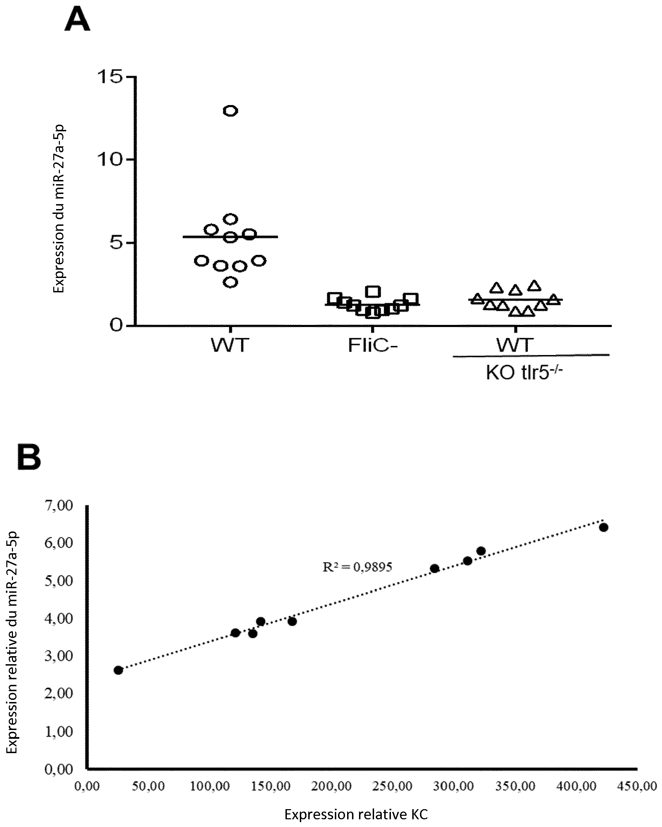

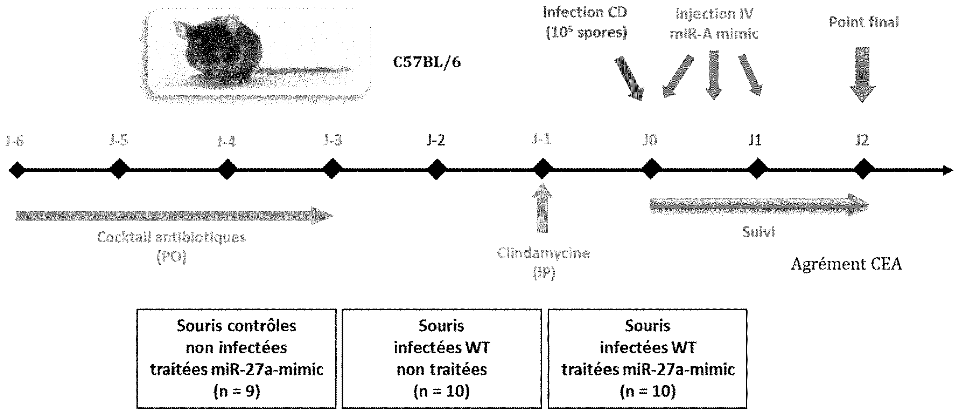

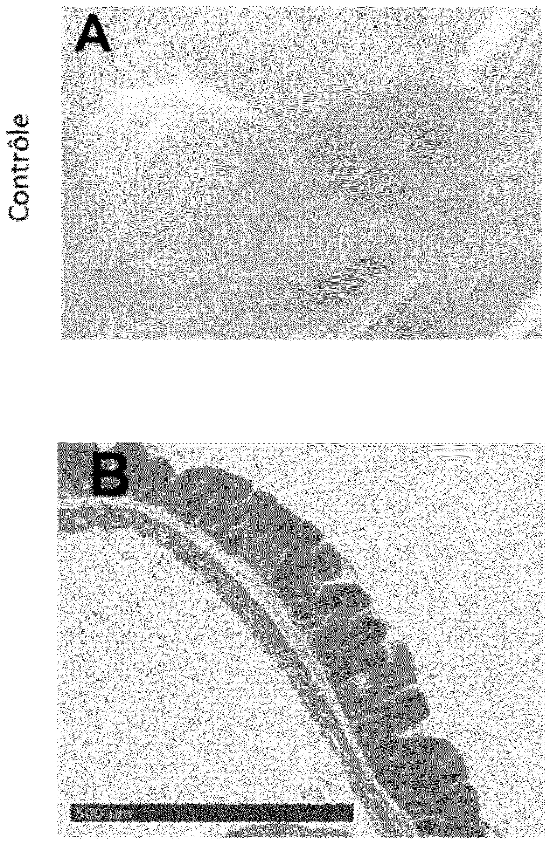

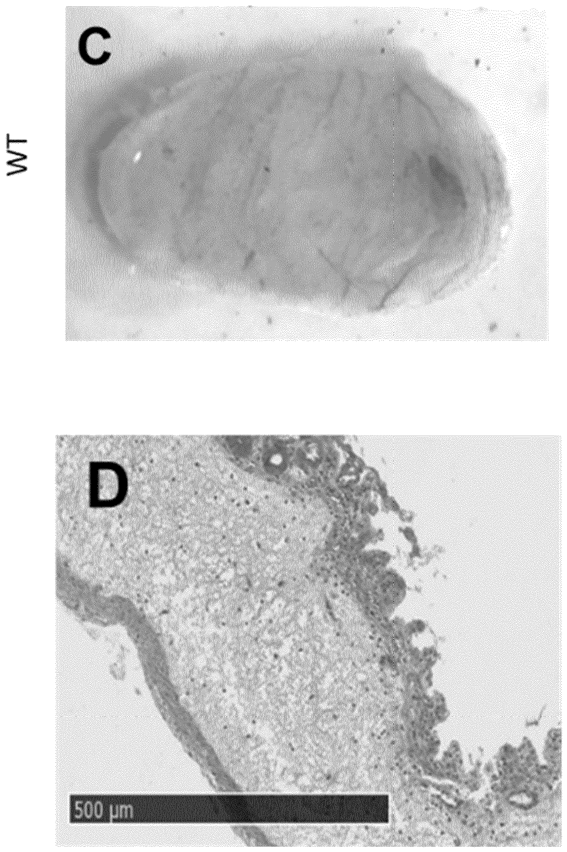

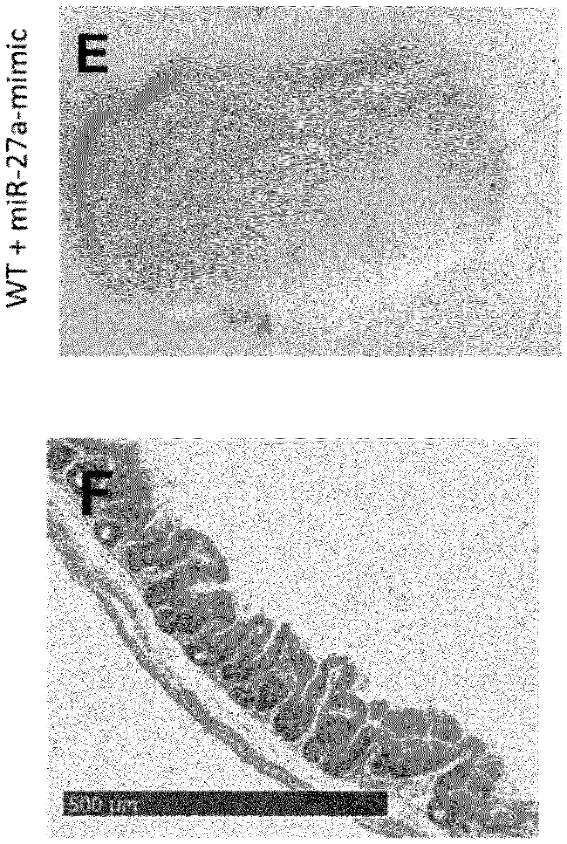

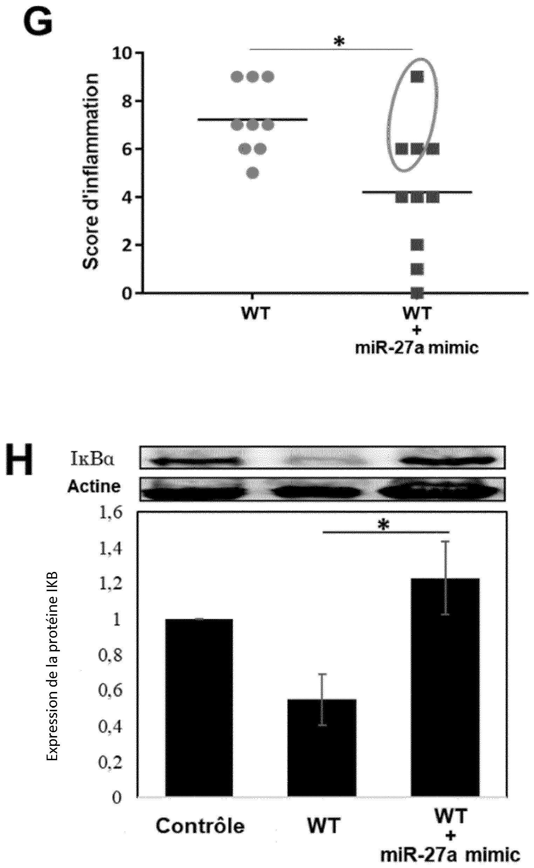

Le miRNA connu dans la littérature sous le nom de « miR-27a-5p » est apparu comme étant significativement surexprimé au cours du temps dans des cellules Caco-2 stimulées par les flagelles de C. *difficile* ou infectées par la bactérie entière (Figure 1). Ces résultats montrent que ce miRNA joue un rôle dans la réponse induite par les flagelles de *C*. *difficile.* Par ailleurs, l'analyse de l'expression *in vivo* des miR-27a-5p dans le caecum de souris à microbiote conventionnel ou des souris KO pour le TLR5 *(tlr5⁻*^{/}*⁻)* infectées par *C*. *difficile* ou un mutant non flagellé, a révélé que ce miRNA est également surexprimé dans le caecum de souris infectées par la bactérie sauvage, alors qu'aucune variation dans l'expression de ce miRNA n'a été observée dans le caecum des souris infectées par un mutant non flagellé ni dans le caecum des souris KO *tlr5⁻*^{/}*⁻* infectées par la souche sauvage (figure 4). Enfin, un mimique de ce miRNA (« miR-27a-5p-mimic ») a été administré par voie intraveineuse dans un modèle murin d'ICD dans le but d'étudier la réponse de l'hôte, responsable des lésions tissulaires et des manifestations cliniques sévères. Les résultats de cette expérience montrent que le mimique du miR-27a-5p réduit considérablement les manifestations cliniques et anatomopathologiques provoquées par la bactérie lors de l'infection dans ce modèle murin (figures 5 et 6). Ce résultat apporte la preuve du concept du rôle important de ce miR-27a-5p dans la modulation de la réponse inflammatoire au cours de I'ICD.

Plus précisément, l'ensemble des résultats présentés ci-dessous démontrent que les flagelles de C. *difficile* provoquent une surexpression du miR-27a-5p qui diminue le degré de la réponse inflammatoire et réduit en conséquence les manifestations cliniques observées durant l'infection intestinale par C. *difficile.*

Ces travaux mettent en lumière le rôle capital du miR-27a-5p pour diminuer la réponse inflammatoire induite par les toxines et les flagelles de *C. difficile* et prévenir (ou diminuer) les lésions muqueuses causées par cette inflammation. Ce miRNA impacte donc la présentation clinique ainsi que le pronostic de I'ICD.

Pour diminuer les effets délétères sévères de l'infection par cette bactérie, les présents inventeurs proposent d'utiliser ce miRNA en tant que principe actif dans une composition thérapeutique.

Dans un premier aspect, la présente invention vise une composition pharmaceutique contenant un mimique du miR-27a-5p, pour son utilisation pour prévenir ou diminuer les lésions intestinales chez un sujet souffrant d'une infection à *Clostridium difficile.*

Dans le cadre de la présente demande, le terme « miRNA » est utilisé pour désigner indifféremment un micro-ARN (ou miARN) ou un « micro-RNA ». Il s'agit de courts ARN non-codants possédant entre 20 et 25 nucléotides, se liant à des ARNm cibles et inhibant ainsi leur traduction. Ces miRNAs peuvent être sous forme double-brin (précurseur ou duplex) ou simple-brin (forme mature, prête à se lier à l'ARNm cible).

Le miRNA connu sous la dénomination « miR-27a-5p » est un ARN double-brin ayant chez la souris et l'homme la même séquence SEQ ID NO :1 (AGGGCUUAGCUGCUUGUGAGCA). Il a été identifié en 2001 ([41]) et a été impliqué dans la voie NF-κB dans des cellules aortiques ([42]), et dans le cancer ([43]). Il a également été identifié dans les cellules interstitielles de la valve mitrale ([46]).

Les mécanismes cellulaires mis en jeu dans ces différentes études sont totalement décorrélés des résultats observés par les présents inventeurs : dans les deux articles [42] et [46], les récepteurs TLRs ne sont pas impliqués. Dans l'article [46], la voie de NF-κb n'est pas activée, c'est la voie de JNK et Wnt/B-caténine qui est impliquée.

En outre, il est bien connu dans l'art qu'un même miRNA peut avoir un rôle donné dans des conditions et dans un type cellulaire donnés et un rôle complétement opposé dans un autre type de cellule. Par exemple, le miR-27a lui-même peut avoir un rôle oncogénique dans le cancer du sein, mais suppresseur de tumeur dans les cancers de la prostate ([47] ; [48]). A l'inverse des présents inventeurs, certaines équipes ont même suggéré que le miR-27a augmentait la réponse inflammatoire médiée par IL-10, dans des macrophages activés ([49]). Il est également connu que le miR-146a a des effets opposés selon les cellules considérées (effet pro-inflammatoire dans les cellules endothéliales stimulées par des surnageants de monocytes activés au LPS [50] ou effet anti-inflammatoire dans les cellules endothéliales vasculaires stimulées au LPS, [51]).

Par « mimique du miR-27a-5p », on entend ici toute molécule synthétique d'ARN double-brin qui a la même séquence ou une séquence similaire à celle de miR-27a-5p permettant de moduler la même cible génétique. De préférence, cette molécule synthétique diffère des miRNAs naturels en ce qu'elle a subi des modifications chimiques visant à augmenter sa stabilité vis-à-vis notamment des RNases et des protéines du complexe RISC (« RNA-induced silencing complex »).

Lesdites modifications chimiques sont par exemple la méthylation, l'addition de groupes phosphorothioates (remplacement de l'oxygène non-liant du groupe phosphate par un atome de soufre), l'ajout de groupements méthyles (par exemple 2'-O-methyl ou 2'-O-methoxyethyl) à différents endroits de la structure ([44])).

Par « séquence similaire », on entend ici une séquence d'acide nucléique ayant un pourcentage d'identité supérieur ou égal à 70 %, de préférence supérieure ou égale à 80 %, de manière encore plus préférée supérieure ou égale à 90 %, avec la séquence du miR-27a-5p naturelle connue (par exemple SEQ ID NO :1). Par « pourcentage d'identité » entre deux séquences d'acide nucléique, on entend désigner un pourcentage de nucléotides identiques entre les deux séquences à comparer, obtenu après le meilleur alignement (alignement optimal), ce pourcentage étant purement statistique et les différences entre les deux séquences étant réparties au hasard et sur toute leur longueur.

Ce pourcentage d'identité peut être calculé par toute méthode d'analyse de séquences bien connue de l'homme du métier. Le pourcentage d'identité peut être déterminé après alignement global des séquences à comparer prises dans leur intégralité. Outre manuellement, il est possible de le déterminer au moyen de l'algorithme de Needleman et Wunsch (1970). Pour les séquences nucléotidiques, la comparaison des séquences peut être effectuée à l'aide de tout logiciel bien connu de l'homme du métier, comme par exemple le logiciel Needle. Les paramètres utilisés peuvent notamment être les suivants : « Gap Open » égal à 10,0, « Gap Extend » égal à 0,5 et la matrice EDNAFULL (Version EMBOSS du NCBI NUC4.4). Ce pourcentage peut également être déterminé après alignement local des séquences à comparer. Outre manuellement, il est possible d'utiliser l'algorithme de Smith et Waterman (1981) pour déterminer ce pourcentage. De préférence, le pourcentage d'identité défini dans le cadre de la présente invention est déterminé au moyen d'un alignement global des séquences à comparer sur toute leur longueur.

De préférence, le « mimique du miR-27a-5p » est une molécule synthétique d'ARN double-brin qui contient au moins 5 ou 6 nucléotides consécutifs de miR-27a-5p [partie qui se lie au mRNA cible]. Le reste de sa séquence oligonucléotidique a alors de préférence un pourcentage d'identité avec l'autre partie du miRNA de 70 % au moins.

De manière encore plus préférée, ledit « mimique du miR-27a-5p » est l'ARN double-brin ayant pour séquence SEQ ID NO :2 (5'-AGGGCUUAGCUGCUUGUGACCCCC-3') et ayant pour séquence accompagnante SEQ ID NO :3 (5'-GGGGGUCACAAGCAGCUAAGCCCU-3') (ou son complémentaire), commercialisée par RIBOXX sous le numéro M-00303-0200 Mimétique CONmiR^{®} in vivo.

Dans la composition pharmaceutique de l'invention, le miARN mimique peut être nu. Il peut également être complexé avec des molécules chimiques ou des nanoparticules (i.e., des particules ayant un diamètre inférieur à 1 µm) chargées positivement, ou encore être enveloppé dans des matériaux protecteurs, par exemple des capsules, des liposomes, etc. de sorte à favoriser sa stabilité et sa transfectabilité (entrée dans les cellules cibles).

Tout moyen de stabilisation connu pour favoriser le transport et le ciblage des siRNAs pourra être utilisé pour faciliter l'administration du mimique de l'invention, car ces molécules ont la même structure. Ces moyens sont, par exemple, ceux décrits dans [40] et [45].

Dans un mode de réalisation préféré, ledit mimique du miR-27a-5p est donc associé à (ou inclus dans) un vecteur, une matrice ou des particules favorisant sa stabilité et sa transfectabilité.

S'il est véhiculé par un vecteur, ledit vecteur est de préférence un virus recombinant choisi parmi : les adénovirus, les rétrovirus, les lentivirus, les virus adéno-associé (AAV), les virus de l'herpès, les cytomégalovirus (CMV), les virus de la vaccine, etc.

De manière avantageuse, ledit virus recombinant est un virus défectif, par exemple un AAV défectif.

Le terme « virus défectif » désigne ici un virus incapable de se répliquer dans une cellule cible. Généralement, le génome des virus défectifs est dénué d'au moins une des séquences nécessaires pour la réplication dudit virus dans la cellule infectée. Ces régions peuvent être soit éliminées, soit rendues non fonctionnelles ou encore substituées par d'autres séquences et en particulier par l'acide nucléique qui code le peptide d'intérêt. Néanmoins, de préférence, le virus défectif conserve malgré tout les séquences de son génome qui sont nécessaires pour l'encapsulation des particules virales.

Ce vecteur peut également être un bactériophage, ceux-ci ayant été déjà utilisés avec succès pour véhiculer des siRNA. Les bactériophages tels que MS2, Phi29, etc. peuvent être utilisés dans le cadre de la présente invention.

Si ledit mimique du miR-27a-5p est associé à des particules, celles-ci peuvent être celles déjà proposées pour le transport des siRNAs. Il peut s'agir par exemple de liposomes (DOPC), de nanoparticules lipidiques, de nanocellules (type EnGenelC EDV), de nanoparticules de silice, d'exosomes, etc.

Plus précisément, ces particules peuvent être des nanoparticules de poly(alkylcyanoacrylate), de préférence recouvertes de chitosan. Le miRNA mimique de l'invention pourra y être aisément adsorbé par formation d'une paire d'ion, comme démontré précédemment pour les siRNA [38].

Si ledit mimique du miR-27a-5p est inclus dans une matrice, ladite matrice peut être une émulsion lipidique neutre (NLE), du polyethylenimine (PEI), du poly(lactide-co-glycolide) (PLGA), etc.

Plus précisément, ledit mimique du miR-27a-5p peut être complexé dans un polymère cationique tel que le « in vivo-jet PEI^{®} », bien connu pour transporter efficacement tout type d'acides nucléiques *in vivo* dans différents organes, sans susciter de réponse inflammatoire.

Il est également possible d'administrer le mimique de l'invention conjugué avec une protéine ou une molécule chimique d'intérêt, par exemple choisie parmi : les lipides, le cholesterol, le PEG, la cyclodextrine, le chitosan, les dendrimères (de poly(amidoamine) ou de poly(propylenimine), le N-acetyl-D-galactosamine (GaINAc), etc.

La présente invention vise également des méthodes pour traiter des sujets souffrant d'une infection à *Clostridium difficile.* Ces méthodes contiennent une étape d'administration de la composition pharmaceutique de l'invention à des patients ayant été infectés par *Clostridium difficile.*

La présente invention concerne enfin l'utilisation du mimique de l'invention, tel que défini ci-dessus, pour fabriquer un médicament destiné à traiter des patients ayant été infectés par *Clostridium difficile.*

Le diagnostic de I'ICD se fait classiquement par i) la suspicion devant un tableau clinique de diarrhée, de colite avec présence de mucus et du sang dans les selles, le développement de complications comme le mégacôlon, la colite pseudomembraneuse, la perforation intestinale, ou ii) la mise en évidence dans les selles des toxines ou de la bactérie elle-même (culture microbiologique, PCR, test GDH).

Un test de cytotoxicité permet de détecter la présence de toxines libres, tandis qu'une culture toxigénique permet de mettre en évidence la présence de bactéries. Des méthodes standardisées (tests immuno-enzymatiques, PCR, amplifications isothermiques) sont aujourd'hui disponibles pour détecter cette infection rapidement en cas de symptômes (diarrhée).

Dans un mode de réalisation particulier, les sujets bénéficiant du traitement de l'invention sont les sujets présentant une des formes modérées à sévères d'ICD. Par exemple, il s'agit des sujets présentant des diarrhées importantes et persistantes (plus de 3 jours) avec déshydratation, des formes dysentériformes (avec des douleurs abdominales et la présence de sang et de mucus dans les selles), des formes compliquées par des dilatations du côlon (mégacôlon) et des colites pseudomembraneuses (souvent détectée par endoscopie). Il peut également s'agir de patients présentant des comorbidités (maladies cardiaques, diabète, pathologies hépato-digestives, pathologies chirurgicales, etc.) où I'ICD se manifeste souvent sous des formes sévères.

Ces sujets peuvent être des animaux (domestiques ou non, par exemple le chat, le chien, les équidés) ou des êtres humains. De préférence, ces sujets ne sont pas des enfants en bas âge.

Par « traitement », on entend ici un traitement à titre curatif (visant à au moins soulager ou stopper le développement des lésions intestinales induites par les bactéries) ou prophylactique (visant à réduire le risque d'apparition de ces lésions).

La composition pharmaceutique utilisée dans l'invention contient, en tant que principe actif, l'acide nucléique décrit ci-dessus (éventuellement associé ou protégé par des particules, vecteurs, lipides, etc.). Elle contient également, de préférence, un excipient pharmaceutiquement acceptable.

Dans la présente description, on entend désigner par « excipient pharmaceutiquement acceptable », un composé (ou une combinaison de composés) entrant dans une composition pharmaceutique, mais ne provoquant pas de réactions secondaires et qui permet par exemple la facilitation de l'administration du ou des composés actifs, l'augmentation de sa durée de vie et/ou de son efficacité dans l'organisme, l'augmentation de sa solubilité en solution ou encore l'amélioration de sa conservation. Ces excipients pharmaceutiquement acceptables sont bien connus et seront adaptés par l'homme de l'art en fonction de la nature et du mode d'administration du ou des composés actifs choisis.

De préférence, les mimiques de l'invention seront administrés par voie systémique, en particulier par voie intraveineuse, par voie intramusculaire, intradermique, intrapéritonéale ou sous-cutanée, ou par voie orale. De manière plus préférée, ils seront administrés à plusieurs reprises, de manière étalée dans le temps.

Leurs modes d'administration, posologies et formes galéniques optimaux peuvent être déterminés selon les critères généralement pris en compte dans l'établissement d'un traitement adapté à un patient comme par exemple l'âge ou le poids corporel du patient, la gravité de son état général, la tolérance au traitement et les effets secondaires constatés.

Les principes actifs peuvent être formulés également sous forme de microcapsules, éventuellement avec un ou plusieurs supports additifs. Pour la préparation de ces microcapsules, les principes actifs sont en général combinés à des diluants appropriés, des stabilisants appropriés, des agents favorisant la libération prolongée des substances actives ou tout autre type d'additif pour la formation d'un noyau central qui est ensuite revêtu d'un polymère approprié (par exemple une résine hydrosoluble ou une résine insoluble dans l'eau). Les techniques connues de l'homme du métier seront utilisées à cet effet. Les microcapsules ainsi obtenues sont ensuite éventuellement formulées dans des unités de dosage appropriées.

Les mimiques de l'invention peuvent également être formulés dans des liposomes. Les liposomes sont formés à partir de phospholipides qui sont dispersés dans un milieu aqueux et forment spontanément des vésicules bicouches concentriques multilamellaires. Ces vésicules ont généralement un diamètre de 25 nm à 4 pm et peuvent être soniquées, conduisant à la formation de vésicules plus petites unilamellaires, de diamètre de 200 à 500 Å, contenant une solution aqueuse en leur cœur. Les liposomes seront particulièrement avantageux pour administrer le médicament à une cible cellulaire ou tissulaire précise. Pour cela, les lipides pourront être couplés chimiquement à des molécules de ciblage, tels que des peptides de ciblage (par exemple hormones), ou des anticorps.

La posologie dépend naturellement du vecteur / complexe considéré, du mode d'administration, du stade de l'infection, de l'âge du patient et de son état. Certains auteurs ont utilisé des doses de miRNAs de 10 mg/kg par voie intraveineuse. Des doses moins importantes peuvent également être utilisées (par exemple, entre 3 et 5 mg/kg par jour).

Lorsque le mimique de l'invention est associé à un polymère cationique ou à un autre vecteur, il est possible d'utiliser des doses plus fortes (25mg/kg), en une prise, ou en plusieurs fois, en répétant les injections.

### Légende des figures

**Figure 1****. Cinétique d'expression du miR-27a-5p par les cellules Caco-2 incubées avec la flagelline FliC ou *C. difficile.* (A)** Cinétique d'expression du miR-27a-5p par les cellules Caco-2 incubées avec FliC durant 12 h. **(B)** Les cellules ont été incubées avec FliC ou infectées par la souche sauvage R20291 (WT) ou son mutant non flagellé (FliC-) pendant 1 h. L'expression du miR-27a-5p a été mesurée par qRT-PCR avec l'oligonucléotide du Tableau 1. Les barres sur chaque point correspondent aux écart-types des moyennes d'au moins 3 expériences indépendantes.

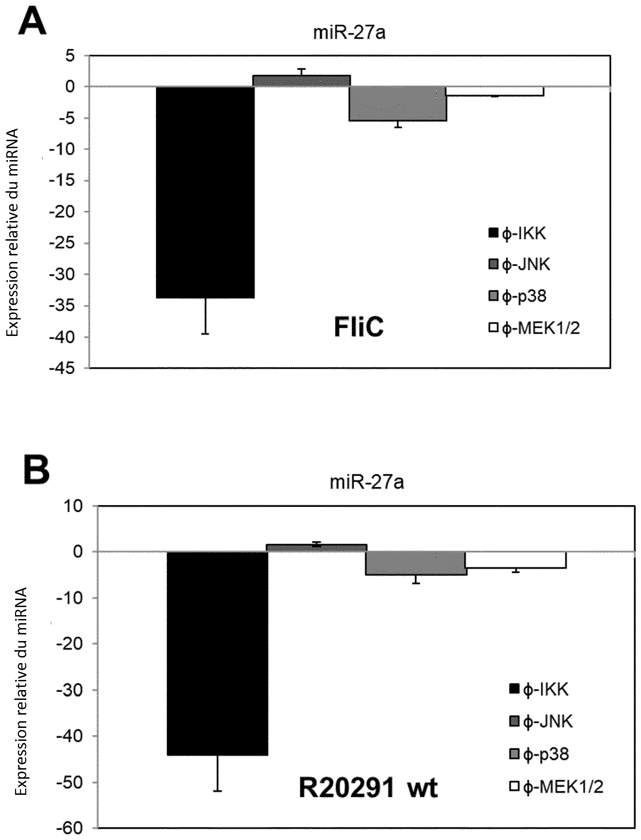

**Figure 2****. Inhibition de la surexpression du miR-27a-5p par des inhibiteurs chimiques spécifiques de NF-kB et des MAPKs dans les cellules Caco-2 incubées avec la flagelline FliC ou la souche R20291 de C. *difficile.*** Les voies NF-κB et MAPKs ont été inhibées 1 h avant la stimulation ou l'infection des cellules, par 20 µM BMS 345541 (inhibiteur IKKα), 20 µM U0126 (inhibiteur MEK1/2), 20 µM PD98059 (inhibiteur MEK1), 20 µM SP600125 (inhibiteur JNK) ou 3 µM SB203580 (inhibiteur p38) aux monocouches cellulaires. L'expression du miR-27a-5p a été analysée après incubation des cellules avec FliC **(A)** ou infection par la souche sauvage R20291 (WT) **(B)** pendant 1 h. L'expression des miRNAs a été mesurée par qRT-PCR avec l'oligonucléotide du Tableau 1. Les barres sur chaque point correspondent aux écart-types des moyennes d'au moins 3 expériences indépendantes.

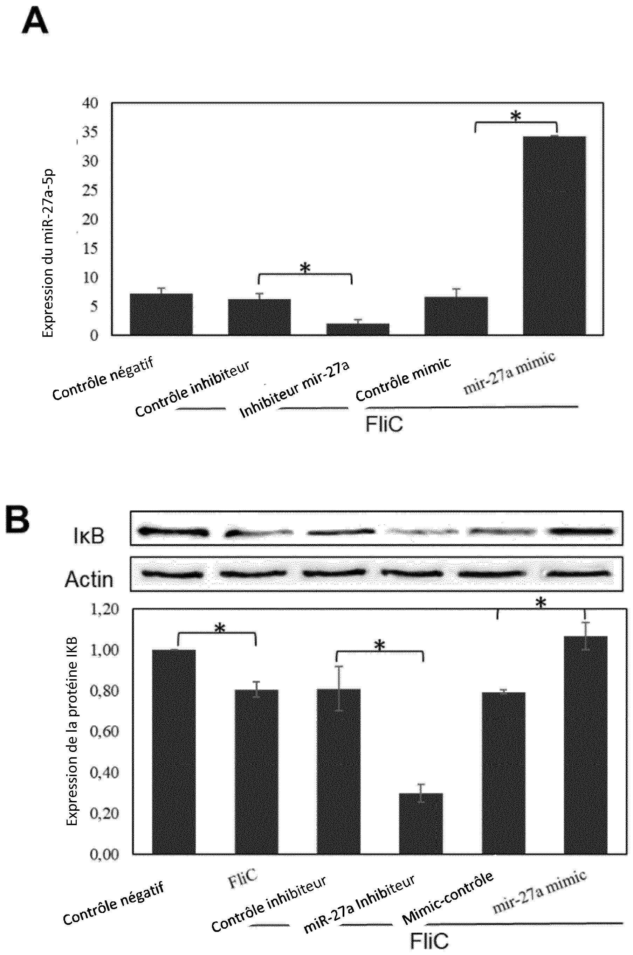

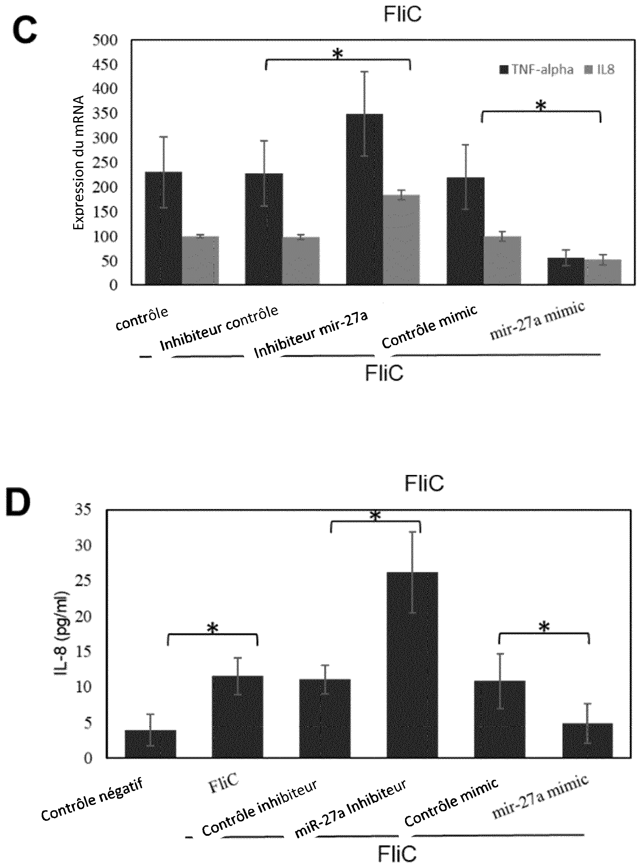

**Figure 3****. Effet inhibiteur du miR-27a-5p sur la voie de NF-κB dans les cellules Caco-2 incubées avec la flagelline.** Les cellules Caco-2 ont été transfectées par les respectifs miR-27a-mimic, miR-27a inhibitor, ainsi que par les respectifs oligonucléotides mimic et inhibitor contrôles. L'expression du miR-27a-5p a été mesurée par qRT-PCR avec l'oligonucléotide 5'-AGGGCTTAGCTGCTTGTGAG-3' (SEQ ID NO :4). **(A).** La dégradation de l'effecteur IκB-α signant l'activation de la voie NF-κB a été mesurée par Western-blot **(B). (C)** L'expression des ARNm des cytokines pro-inflammatoires TNFα et IL-8 (oligonucléotides Tableau 2), ainsi que **(D)** la production d'IL-8 dans le surnageant de culture cellulaire par ELISA a été également mesurée. Les barres sur chaque point correspondent aux moyennes ± écart-types, pour au moins 3 expériences indépendantes (^{∗} p<0.01).
**Figure 4****. Expression du miR-27a-5p dans le caecum des souris infectées par C. *difficile.* (A)** Expression du mir-27a-5p mesurée par qPCR dans le caecum des souris à microbiote conventionnel infectées par la souche sauvage R20291 (WT) ou par le mutant isogénique non flagellé (FliC-). Le troisième groupe correspond à l'expression du mir-27a-5p chez les souris KO *tlr5*^{*-*/*-*} infectées par la souche WT. Les barres horizontales correspondent aux moyennes par groupe. **(B)** Corrélation de l'expression du mir-27a-5p avec l'expression de l'ARNm de KC (équivalent de l'IL-8 humaine, oligonucléotides Tableau 2) dans le caecum des souris conventionnelles infectées par la souche R20291 WT.
**Figure 5****. Protocole de traitement par le miR-27a-5p-mimic sur le modèle murin d'ICD.** Des groupes de souris C57BL/6 à microbiote conventionnel femelles âgées de 4 à 5 semaines sont prétraitées par un cocktail d'antibiotiques ajouté à l'eau de boisson de J-6 à J-3 avant l'infection J0. (0,4 mg/mL kanamycine, 0,035 mg/mL gentamycine, 850 U/mL colistine, 0,215 mg/mL métronidazole et 0,045 mg/mL vancomycine). A J-1, 0,2 mL d'une solution de clindamycine (1,25 mg/mL), sont injectés par voie intra-péritonéale (IP). A J0, les souris sont infectées par gavage oral avec 0,5 mL d'une suspension contenant 10⁵ spores de la souche sauvage (0,5 mL d'eau stérile pour souris témoins). Les souris des groupes traités reçoivent ensuite la suspension miR-27a-5p mimic / in vivo-jetPEl^{®} à raison de 3 doses de 50 µg (soit 25 mg/kg), au moment de l'infection, 8 h et 24 h après l'infection, par voie intra-veineuse (150 µL injectés en rétro-orbital). L'état clinique des animaux est observé et à J2, les souris sont sacrifiées pour l'analyse des caecums.
**Figure 6****. Inflammation caecale chez les souris infectées par C. *difficile.*** Des groupes de souris C57BL/6 à microbiote conventionnel (n = 9-10) ont été infectées et traitées ou pas par le miR-27a-mimic. Les caecums ont été collectés à J2 post-infection. Caecum entier et coupe histologique représentatifs **(A, B)** d'une souris contrôle (non infectée et traitée par le miR-27a-mimic), **(C, D)** d'une souris infectée par la souche sauvage R20291 WT et **(E, F)** d'une souris infectée par la même souche et traitée par le miR-27a-mimic. **(G)** Scores histologiques d'inflammation (Tableau 3) pour les souris individuelles de chaque groupe. Les lignes horizontales représentent les moyennes des scores pour chaque groupe (score 0 pour le groupe contrôle non représenté). Le cercle rouge indique les 3 animaux ayant présenté des manifestations d'ICD. **(H)** Western blots réalisés à partir des lysats de caecums révélé à l'aides d'un anticorps dirigé contre IκB-α et l'actine. La photo représente un blot représentatif d'une expérience. La densité des bandes est mesurée à l'aide du logiciel Fusion et les ratios IκB-α/actine ont été calculés. Pour le contrôle négatif, le ratio a été normalisé à 1 et les ratios des autres échantillons ont été reportés au contrôle négatif. Les barres sur chaque point correspondent aux moyennes ± écart-types pour chaque groupe d'animaux. ^{∗} p <0.05.

### Exemples

### Matériel et méthodes :

### I. Souches bactériennes

Deux souches de C. *difficile* ont été utilisées dans l'infection des modèles animaux : la souche sauvage R20291 (NAP1/027) et son mutant isogénique, dont le gène *fliC* de la flagelline est inactivé (ΔFliC). Cette souche mutée a été construite par la technologie du ClosTron (N. Minton, Université de Nottingham) qui consiste en une inactivation insertionelle des gènes **[22].** Ces deux souches sont conservées, sous forme de formes végétatives ou de spores, congelés à -80°C.

### II. Purification de la protéine recombinante FliC de C. difficile par chromatographie d'affinité

La flagelline FliC de la souche R20291 (NAP1/027) de C. *difficile* a été produite chez une souche *d'Escherichia coli* BL21 hébergeant le plasmide pET28 qui code la protéine FliC avec une queue histidine à l'extrémité N-terminale (FliC-His), préalablement construit au laboratoire.

Une préculture d'E. *coli* BL21 pET28 en milieu LB (Luria Bertani), contenant 50 µg/mL de kanamycine (permettant le maintien du plasmide) est diluée au 1/100^{ème} dans 2 L du même milieu et incubée ensuite à 37°C pendant 3 h. L'induction de la synthèse de FliC-His est ensuite effectuée par l'ajout d'IsoPropylThioGalactoside (IPTG) à 1 mM. Après 3 h d'incubation à 37°C, la culture est centrifugée 15 min à 4°C à 4 000 rpm. Le culot est ensuite repris dans 50 mL de tampon de lyse contenant 25 mM de Tris HCl pH 8, 300 mM de NaCl, 1 mg/mL de lysozyme (Sigma) et 500 µl d'inhibiteur de protéase P-8465 (Sigma). La lyse est effectuée à 4°C pendant 1 h sous agitation. Trois cycles de congélation-décongélation à -80°C puis 5 cycles de sonication de 45 s chacun sont ensuite effectués pour lyser les bactéries. La suspension est centrifugée ensuite 40 min à 10 000 g à 4°C. Le culot est repris dans 10 mL de tampon d'équilibration contenant 50 mM de NaH₂PO₄, 8 M d'urée et 300 mM de NaCl. La suspension est centrifugée 40 min à 10 000 g et le surnageant est récupéré et filtré sur filtre 0,45 µm puis mis en contact pendant 1 h à 4°C avec 2 mL de résine chargée en cations divalents Co++ (Talon^{®}, Clontech) lavée préalablement par 2 volumes d'eau ultrapure (Millipore^{®}) et 3 volumes de tampon d'équilibration. Ensuite le mélange est déposé sur la colonne, la résine est lavée 3 fois par le tampon d'équilibration et la protéine FliC-His est ensuite éluée par 3 mL de tampon d'élution contenant 45 mM de NaH₂PO₄, 8 M d'urée, 270 mM de NaCl et 150 mM d'Imidazole. L'éluat est ensuite dialysé contre du PBS à 4°C à l'aide d'une cassette de dialyse Slide-A-Lyser^{®} (Thermo Scientific) permettant une dialyse des molécules de masse moléculaire inférieure à 10 000 Da.

Après dialyse, une électrophorèse en gel de polyacrylamide à 12 % (SDS-PAGE), suivie d'une coloration au bleu de Coomassie 0,25 % a été effectuée pour vérifier la pureté de l'échantillon. Une gamme-étalon de BSA (Albumine Sérique Bovine) fraction V (Euromedex^{®}) est également déposée sur gel et l'établissement de la courbe d'étalonnage est réalisé grâce au logiciel ImageJ par mesure de la densité des bandes. Enfin, la concentration de FliC-His est déterminée par mesure de la densité de la bande correspondant à la protéine recombinante, après dépôt de 10 µL d'éluat et calculée grâce à l'équation de la courbe d'étalonnage.

### III. Culture cellulaire

La lignée cellulaire Caco-2 (adénocarcinome colique) a été utilisée. Il s'agit d'un des modèles cellulaires les plus employés pour modéliser *in vitro* l'épithélium intestinal. Les cellules Caco-2 sont des cellules épithéliales intestinales humaines qui expriment plusieurs TLRs, dont le TLR5. La lignée est cultivée en milieu DMEM^{®} (Dulbecco's Modified Eagle Médium, Gibco) additionné de sérum de veau fœtal décomplémenté (SVF) à 15 % et 1 % d'acides aminés non essentiels (Gibco).

La lignée cellulaire est stockée dans des ampoules de congélation à l'azote liquide à -120°C dans du SVF additionné de 10 % de DMSO (cryoprotecteur). Après décongélation, les cultures sont réalisées dans des flasques de 25 et 75 cm² et deux repiquages sont effectués lorsque les cellules sont confluentes. Les cellules sont comptées à l'aide d'une cellule de Malassez, puis distribuées dans de nouvelles boîtes. Les cellules en culture sont maintenues dans un incubateur à 37°C sous une atmosphère contenant 10 % de CO₂ et saturée en vapeur d'eau. Le milieu est changé 48 h après la décongélation puis tous les jours. La recherche de contamination par des mycoplasmes est effectuée en fixant les tapis cellulaires au fixateur de Carnoy puis en les colorant au réactif de Hoechst (Sigma) dilué au 1/2000^{e}. Les tapis cellulaires sont alors observés en microscopie à fluorescence.

### IV. Infection et traitement des cellules

Les cellules Caco-2 sont mises en contact avec la protéine recombinante FliC-His purifiée de *C. difficile* R20291 ou avec les souches de C. *difficile.* Des plaques de 24 puits sont ensemencées avec 5×10⁵ cellules par puits et les cellules sont ensuite cultivées jusqu'à 3 jours post-confluence, avant qu'elles atteignent la polarisation. Les cellules sont déplétées 4 h avant le contact avec FliC ou l'infection par les différentes souches de C. *difficile* afin d'éviter l'activation des voies de signalisation, par lavage par PBS puis addition de 0,5 mL de milieu DMEM additionné de 1 % de SVF au lieu de 15 %. Ensuite, les monocouches cellulaires sont mises en contact avec la protéine FliC-His à une concentration de 10 ng/mL avec des temps d'incubation de 1, 2, 4, 6, 8, 12 et 24 h. Les cellules Caco-2 non mises en contact avec FliC-His constituent le témoin négatif pour chaque temps d'étude. À la fin de la cinétique d'incubation, le milieu de culture de chaque puits est aspiré et les cellules sont directement lysées pour l'extraction des ARN. Chaque expérience est réalisée au moins trois fois de manière indépendante.

Pour l'infection des cellules par C. *difficile,* la souche R20291 sauvage ou la souche mutant non flagellée de C. *difficile* sont cultivées préalablement en milieu BHI liquide en chambre anaérobie à 37°C. La suspension bactérienne est centrifugée à 5000 rpm pendant 4 min, puis lavée avec 10 mL de PBS. Après, une nouvelle centrifugation à 5000 rpm pendant 4 min, le culot est repris dans 500 µL de PBS, soit une suspension bactérienne de 2×10⁹ bactéries/mL. 250 µL de suspension bactérienne sont mis en contact avec les cellules, soit une MOI *(Multiplicity oƒ Infection)* de 50 bactéries/cellules.

Lorsque cela est indiqué, les voies NF-κB et MAPKs ont été inhibées chimiquement 1 h avant la stimulation ou l'infection des cellules, en ajoutant aux monocouches cellulaires 20 µM BMS 345541 (inhibiteur IKKα) (Sigma), 20 µM U0126 (inhibiteur MEK1 / 2), 20 µM PD98059 (inhibiteur MEK1), 20 µM SP600125 (inhibiteur JNK) ou 3 µM SB203580 (inhibiteur p38) (Cell Signaling, Ozyme).

### V. Surexpression et inhibition du miR-27a-5p dans les cellules Caco-2

Le miR-27a-5p a été surexprimé dans les cellules Caco-2 en culture en utilisant 25 nM du mimique Syn-hsa-miR-27a-5p, alors que l'inhibition a été réalisée avec 250 nM d'Anti-hsa-miR-27a-5p, tous deux achetés auprès de Qiagen. Avant la transfection, les cellules Caco-2 ont été ensemencées dans une plaque de 24 puits à raison de 6 x 10⁴ cellules/puits et transfectées avec le réactif de transfection HiPerfect (Qiagen) selon les instructions du fabricant. Les cellules ont été incubées avec des complexes de transfection dans leurs conditions de croissance normales et récoltées 48 h après la transfection. Les *miScript Inhibitor Negative Control* et *miScript Negative Control* (Qiagen) ont été respectivement utilisés comme contrôle négatif et positif. Pour surveiller l'efficacité de la transfection, le *AllStars Hs Cell Death Control siRNA* (Qiagen) a été incorporé par transfection dans des cellules en parallèle dans toutes les transfections, et une efficacité de transfection d'au moins 75 % a été atteinte. Lorsque nécessaire, les cellules ont été stimulées par la flagelline FliC purifiée pendant 1 h. Le niveau de miR-27a-5p et l'expression des gènes de l'Interleukin 8 (IL-8) et du TNFα ont été analysés par RT-PCR quantitative (qRT-PCR), le taux de protéine IκB a été évalué par Western blot et le niveau d'IL-8 dans le surnageant cellulaire a été dosé par ELISA.

### VI. Modèle animal

La souris C57BL/6 à microbiote conventionnel a été utilisée comme modèle animal d'infection à *C. difficile* (ICD). Des souris femelles âgées de 4 à 5 semaines (Charles River Laboratoires) sont maintenues en isolateur stérile, alimentées avec de la nourriture irradiée et de l'eau stérilisée par autoclavage. A l'arrivée des animaux, les fèces sont prélevées afin de vérifier l'absence de colonisation intestinale par C. *difficile.*

Dans ce modèle d'infection, les souris ont été prétraitées par un cocktail d'antibiotiques ajouté à l'eau de boisson pendant 4 jours (de J-6 à J-3 avant l'infection J0). Ce cocktail est composé de 0,4 mg/mL de kanamycine, 0,035 mg/mL de gentamycine, 850 U/mL de colistine, 0,215 mg/mL de métronidazole et de 0,045 mg/mL de vancomycine. Ensuite, le traitement a été arrêté deux jours. Un jour avant l'infection (J-1), 0,2 mL d'une solution de clindamycine de 1,25 mg/mL, ont été injectés par voie intra-péritonéale (IP). A J0 (infection), les souris ont été infectées par gavage oral avec 0,5 mL d'une suspension contenant 10⁵ spores de la souche sauvage ou de la souche ΔFliC, alors que le gavage des souris témoins a été effectué avec 0,5 mL d'eau stérile.

La solution de miR-27a-5p mimic utilisée pour le traitement des souris a été préparée en mélangeant le miR-27a-5p mimic (synthétisé par Riboxx^{®}, Allemagne) avec un réactif de transfection *in vivo,* le in vivo-jetPEl^{®} (Polyplus-transfection^{®}, France). Après remise en suspension du miR-27a-5p mimic lyophilisé en eau millipore stérile, 50 µg de miR-27a-5p mimic ont été dilués avec une solution de glucose stérile (concentration finale de glucose de 5 %) ; 6 µL de réactif in vivo-jet PEI ont également été dilués avec une solution de glucose stérile (concentration finale de glucose de 5 %), puis ces deux solutions ont été mélangées volume à volume (concentration finale du miR-27a-5p mimic de 0,33 µg/µL). Les souris appartenant au groupe infecté et traité ont reçu ensuite comme traitement la suspension miR-27a-5p mimic / in vivo-jetPEl^{®} à raison de 3 doses de 50 µg (soit 25 mg/kg), au moment de l'infection, 8 h et 24 h après l'infection, par voie intra-veineuse (150 µL injectés en rétro-orbital).

Suite à l'infection, les souris ont été maintenues en observation pendant 2 jours, et les manifestations cliniques de l'ICD, diarrhée, poil hérissé et activité réduite, ont été répertoriées. Les souris ont été également pesées chaque jour, pour observer les variations de poids potentielles, relatives à une infection.

A J2 post-infection, les souris ont été sacrifiées et le cæcum de chaque souris a été prélevé, coupé longitudinalement pour prélever une quantité suffisante de contenu caecal pour procéder au dénombrement bactérien. Le cæcum a ensuite été lavé en PBS, plongé dans une solution de RNAlater (Qiagen) pour stabiliser les ARN, et découpé en trois parties : une partie pour les études histologiques, une autre pour extraction immédiate des ARN totaux et la dernière pour l'extraction des protéines (pour immuno-blot).

### VII. Dénombrement bactérien

Le contenu cæcal prélevé a été mis en suspension en PBS à 10 mg/mL (solution-mère). Pour le dénombrement des formes végétatives, une gamme de dilution au 1/10^{e} en PBS a été réalisée : 100 (1 mg/mL), 10⁻¹ (0,1 mg/mL), 10⁻² (0,01 mg/mL) et 10⁻³ (0,001 mg/mL). Ainsi, 100 µL de chaque dilution ont été ensemencés sur milieu BHI agar *(Brain Heart Infusion)* additionné de 3 % de sang de cheval défibriné et 0,4 % de supplément Oxoid (125 mg de D-cycloserine et 4 mg de Cefoxitin). Pour le dénombrement des spores, le contenu cæcal a été soumis à un choc alcoolique en incubant la solution-mère avec 1 volume d'éthanol absolu pendant 10 min, afin de tuer les formes végétatives. La suspension ainsi formée a été centrifugée 10 min à 5000 rpm et le culot a été repris dans 1 volume de PBS. Ensuite, une gamme de dilution au 1/10^{e} a été réalisée : 100 (1 mg/mL) et 10⁻¹ (0,1 mg/mL). La solution-mère ainsi que chaque dilution ont été ensemencées sur le même milieu additionné de 0,1 % de Taurocholate (Sigma) permettant la germination et donc le dénombrement des spores de C. *difficile.* Les milieux ont été incubés en chambre à atmosphère contrôlée anaérobie (atmosphère avec 5 % H₂, 5 % CO₂ et 90 % N₂) à 37°C pendant 48 h.

### VIII. Extraction des ARNs

Le kit miRNeasy mini^{®} (Qiagen) a permis l'extraction et la purification des ARN totaux y compris des miRNAs à partir des cultures cellulaires de la lignée Caco-2 et à partir des tissus cæcaux de souris, à la différence de la majorité des kits commerciaux qui ne permettent pas l'extraction des molécules d'ARN dont la taille est inférieure à 200 nucléotides. Ce kit permet une lyse des échantillons basée sur l'utilisation d'un mélange de phénol/guanidine et ensuite une purification des ARN totaux grâce à une colonne de membrane de silice.

Pour l'extraction des ARN des cellules Caco-2, le milieu de culture a été aspiré des puits où 700 µL de QlAzol ont été ensuite ajoutés. Le QlAzol, agent de lyse, est une solution monophasique de phénol et de thiocyanate de guanidine permettant la lyse cellulaire, l'inhibition des RNAses et l'élimination de la majorité de l'ADN et des protéines cellulaires des lysats par extraction organique. La lyse est poursuivie en grattant les tapis cellulaires à l'aide d'un grattoir et en refoulant le lysat plusieurs fois par aller-retour de micropipette. Afin de réduire la viscosité du lysat, les cellules ont ensuite été homogénéisées à l'aide d'une seringue et d'une aiguille en les refoulant dix fois.

Les cæcums prélevés des souris sacrifiées ont été lysés, après l'ajout du QlAzol, à l'aide du FastPrep^{®} permettant l'homogénéisation automatisée des échantillons dans les tubes de Lysing Matrix D^{®} (MP Biomedicals). L'échantillon est broyé de façon mécanique par impacts simultanés et multidirectionnels des billes de Lysing Matrix D^{®} pendant 40 s à la vitesse 6.

Les lysats ont ensuite été incubés à température ambiante pendant 5 min pour permettre la dissociation des complexes nucléoprotéiques. Ensuite, 140 µL de chloroforme ont été ajoutés au tube qui a ensuite été agité vigoureusement pendant 15 s, puis incubé pendant 3 min à température ambiante et suivi d'une centrifugation 15 min à 12 000 g à 4°C permettant la formation de 3 phases : une phase aqueuse transparente contenant les ARN (dans un volume de 350 µL), une interphase blanche contenant l'ADN et une phase organique rouge contenant les protéines. La phase aqueuse a alors été transférée dans un tube collecteur auquel on ajoute 525 µL d'éthanol absolu (1,5 volume) pour assurer les conditions appropriées pour la fixation des ARN sur la colonne. Le mélange ainsi obtenu a été transféré sur une colonne de membrane de silice associée à un tube collecteur. La colonne a été centrifugée pendant 15 s à plus de 8 000 g à température ambiante et le surnageant a été éliminé. Ensuite, 700 µL de tampon RWT^{®} ont été ajoutés à la même colonne qui a été lavée par centrifugation pendant 15 s à plus de 8 000 g, et le surnageant a été éliminé. Deux nouveaux lavages ont ensuite été effectués avec 500 µL de tampon RPE^{®} par centrifugation pendant 15 s à plus de 8 000 g. A la suite du deuxième lavage en tampon RPE^{®}, une centrifugation pendant 2 min à plus de 8 000 g a été effectuée afin de s'assurer de l'absence de l'éthanol durant l'élution de l'ARN. L'élution de l'ARN a été faite par ajout de 40 µL d'eau libre d'ARNase (RNase-free) sur la colonne, suivi d'une centrifugation pendant une minute à plus de 8 000 g.

Enfin, les ARN totaux extraits ont été quantifiés à l'aide d'un spectrophotomètre Nanodrop^{®} (ND-2000) en déposant 2 µL d'échantillon. Cette analyse permet également le calcul du rapport des absorbances à 260 et 280 nm (A260/A280) et celui des absorbances à 260 et 230 nm (A260/A230), permettant d'évaluer la pureté des acides nucléiques présents dans chaque échantillon. L'évaluation de l'intégrité des ARN totaux extraits a été également effectuée par analyse à l'aide du Bioanalyseur Agilent^{®} et mesure de la valeur du RNA Integrity Number (RIN).

### IX. Analyse de l'expression des miRNAs par PCR en temps réel

### IX.1. Synthèse de l'ADN complémentaire (ADNc)

Les miRNAs matures sont naturellement composés d'environ 22 nucléotides et, au contraire des ARNm, ne sont pas poly-adenylés. Pour cette raison, la synthèse de l'ADNc a été effectuée à l'aide du kit miScript RT II^{®} (Qiagen) contenant la reverse transcriptase et la poly(A)-polymérase, un mix d'acides nucléiques (contenant des dNTPs, rATP, amorces oligo-dT et un ARN synthétique servant de contrôle interne), afin d'évaluer la performance de la RT et le tampon « miScript HiSpec » permettant la conversion sélective des miRNAs matures en ADNc. Une poly-adénylation et une transcription inverse ont donc lieu parallèlement dans un même tube. Les miRNAs matures sont poly-adenylés par la poly(A)-polymérase et rétro-transcrits en ADNc utilisant des amorces oligo-dT. Ces amorces possèdent une extrémité 3' dégénérée (3' *degenerate anchor)* et une séquence tag universelle à son extrémité 5' permettant l'amplification des miRNAs matures durant la qPCR. L'association de la poly-adénylation et de l'addition du tag universel assure l'absence de détection d'ADN génomique.

Un mix composé de 4 µL de tampon miScript HiSpec 5X, 2 µL de mix miScript Nucleics 10X et 2 µL de mix « miScript Reverse Transcriptase » a été ajouté à 2 µg d'ARN total et la synthèse de l'ADNc a été effectuée dans un volume final de 20 µL. La réaction est effectuée à 37°C pendant une heure, puis les enzymes ont été inactivées par une incubation de 5 min à 95°C. Les échantillons ont été dilués au 1/10^{e} et conservés à -20°C avant d'être amplifiés par PCR.

### IX.2. PCR quantitative (qPCR)

Les amorces utilisées sont présentées dans le Tableau 1. La PCR a été réalisée avec le kit miScript SYBR^{®} Green PCR (Qiagen^{®}) sur le thermocyleur CFX96 (Bio-Rad). Le kit contient les amorces reverses *(universal primer)* et un master mix contenant l'intercalant (SYBR Green) et l'enzyme HotStarTaq DNA polymerase. Les PCR ont été réalisées dans un volume final de 10 µL, comprenant 1 µL d'une solution diluée de matrice d'ADNc, 9 µL de mélange constitué du réactif de PCR (master mix et les amorces universelles) et des amorces spécifiques de chaque ADNc correspondant aux miRNAs cibles à la concentration finale de 0,5 µM. Le programme thermique inclut, pour toutes les cibles étudiées, une étape initiale d'activation de l'enzyme HotStarTaq DNA polymerase de 15 min à 95°C, puis 40 cycles comprenant chacun une dénaturation de 15 s à 94°C, une hybridation de 30 s à 55°C et une élongation de 30 s à 70°C. L'amplification a été suivie d'une étape de fusion, permettant la détermination de la température de fusion, ou Tm, de chaque amplicon, constituée d'une dénaturation de 1 min à 95°C, d'une réassociation des brins pendant 2 min à 65°C, puis d'une fusion lente par chauffage à 95°C à la vitesse de 0,5°C par seconde.

Les résultats de la PCR sont exprimés en valeur de Ct *(Cycle threshold)* correspondant au nombre de cycles d'amplification nécessaires pour que la fluorescence dépasse le seuil de détection du fluorimètre. Les résultats obtenus ont été exprimés en expression relative.

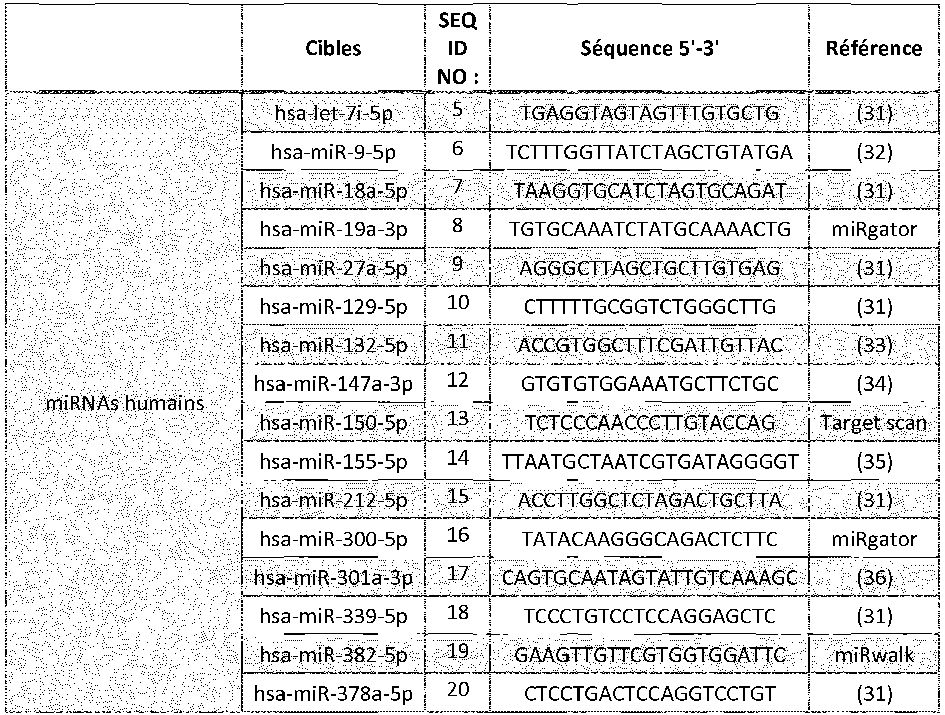

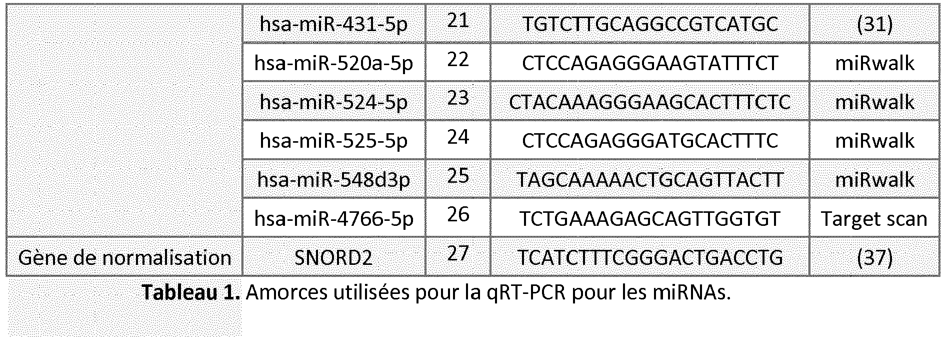

Les qRT-PCR pour l'analyse de l'expression des gènes des cytokines ont été réalisées dans les mêmes conditions à l'aide des oligonucléotides cités dans le Tableau 2 suivant.

**Tableau 2. Oligonucléotides utilisés pour les analyses par qRT-PCR de l'expression des ARNm.**

| **Gène** | **Oligonucléotide** | **SEQ ID NO:** |
|---|---|---|
| hTNF-α | F'-CGAGTGACAAGCCTGTAGCC | 28 |
| | R'-GTTGACCTTGGTCTGGTAGG | 29 |
| hIL-8 | F'-GGCACAAACTTTCAGAGACAG | 30 |
| | R'-AAATTTGGGGTGGAAAGGTT | 31 |
| hACTIN | F'-AGAAAATCTGGCACCACACC | 32 |
| | R'-AGAGGCGTACAGGGATAGCA | 33 |
| mKC (IL-8) | F'-GCTGGGATTCACCTCAAGAA | 34 |
| | R'-AGGTGCCATCAGAGCAGTCT | 35 |
| mGAPDH | F'- GAACTGGCAGAAGAGGCACT | 36 |
| | R'-AGGGTCTGGGCCATAGAACT | 37 |

| | | |
|---|---|---|
| h= human ; m = murin. Les gènes de l'actine et de la GAPDH ont servi à la normalisation | | |

### X. Analyse de l'activation de la voie de signalisation NF-κB par immuno-blot

### X.1. Extraction des protéines

Le morceau de tissu caecal (environ 70 g) a été placé dans 600 µL du tampon de lyse (Tris 62 mM pH 6,8, 10 % Glycérol, SDS 1,5 %, bleu de bromophénol 0,25 %) dans des tubes Lysing Matrix^{®} (MP Biomedicals) contenant des billes de céramique. Les tissus ont été broyés sous agitation mécanique par le FastPrep-24^{®} (MP Biomedicals). Les échantillons ont ensuite été conservés à -20°C avant leur analyse.

### X.2. Electrophorèse des protéines sur gel de polyacrylamide (SDS-Page)

Après dénaturation des échantillons (100°C au bain-marie pendant 10 min), les protéines ont alors été séparées sur gel de polyacrylamide à 12 % en conditions dénaturantes (SDS-PAGE). Huit (8) µL d'échantillons ont été déposés par puits ; 5 µL de marqueur de poids moléculaire (PageRuler^{®} *Plus prestained protein ladder,* ThermoScientific) ont également été déposés sur chaque gel. La migration a été réalisée à voltage constant (200 V) pendant 60 min dans le tampon de migration (Tris 25 mM, SDS 0,1 %, Glycine 192 mM).

### X.3. Transfert et immuno-blot

La membrane de polyvinylidene difluoride (PVDF) a été régénérée avec du méthanol, puis les protéines ont été transférées à ampérage constant à 350 mA pendant 1 h dans le tampon de transfert (Tris 20 mM, Glycine 150 mM). Un immuno-marquage de la protéine IκB-α a ensuite été réalisé puis une révélation par chimiluminescence. Suite au transfert, la membrane a été saturée dans un mélange de TBS (Tris 0.5 M, NaCl 1.5M) additionné de Tween 20 à 0,1 % et de lait 5 % pendant 1 h. Les membranes ont été incubées la nuit à 4 °C avec un premier anticorps (Ac) spécifique dirigé contre la protéine IκB-α (anti-IkB-α, Ac de lapin) ou contre l'actine (Ac de souris) (Cell signaling^{®}). Ces anticorps ont été dilués au 1/2000^{ème} dans du TBS additionné de Tween 20 à 0,1 % et de BSA à 5 %. Ensuite, les membranes ont été lavées 3 fois 10 min dans du TBS additionné de Tween 20 à 0,1 %. Elles ont ensuite été incubées 1 h à température ambiante, sous agitation, avec un 2^{ème} Ac de lapin (IgG anti-rabbit HRP linked antibody, Cell Signaling^{®}) ou de souris (IgG anti-mouse HRP linked antibody, Cell Signaling^{®}) couplés à la peroxydase. Les membranes ont été lavées 3 fois 10 min, puis incubées 1-5 min avec le réactif de révélation Immobilon TM Western Chemiluminescent HRP Substrate^{®}. Les membranes ont été révélées et les images analysées avec l'appareil Fusion^{®} (Viber Lourma), avec une normalisation par l'actine. La densité des bandes a été mesurée à l'aide du logiciel Fusion et les ratios IκB- α/actine ont été calculés. Pour le contrôle négatif, le ratio a été normalisé à 1 et les ratios des autres échantillons ont été reportés au contrôle négatif pour chaque expérience.

### XI. Analyse histologique

Les morceaux de tissu caecal ont été conservés dans une solution de formol pendant 24 heures, puis transférés dans de l'éthanol à 70 %. Les coupes histologiques et la coloration d'Hématoxyline et Eosine ont été réalisées dans la Plateforme d'Histologie souris Immunopathologie de Clamart de l'Institut Paris Saclay d'Innovation Thérapeutique IPSIT (PHIC- 32 rue des carnets, Clamart 92140, France).

L'analyse microscopique des préparations sur lames a été réalisée à l'aide du logiciel NDP View^{®} (Hamamatsu). Un score du degré d'inflammation a été établi sur la base de l'œdème sous-muqueux, l'infiltrat inflammatoire, les lésions épithéliales et la perte des cellules à mucus (Cellules Goblet). Tableau 3.

**Tableau 3. Score d'inflammation utilisé dans l'analyse histologique des caecums.**

| **1. Œdème** | |
|---|---|
| 0 | Absence d'œdème |
| 1 | Œdème minime avec expansion sous-muqueuse multifocale minime (< 2x) |
| 2 | Œdème modéré avec expansion sous-muqueuse multifocale à 2-3x |
| 3 | Œdème sévère avec expansion sous-muqueuse multifocale > 3x |
| 4 | Score 3 avec expansion sous-muqueuse diffuse |

| **2. Infiltrat cellulaire (400x)** | |
|---|---|
| 0 | Absence d'inflammation (0-5 cellules) |
| 1 | Infiltrat inflammatoire neutrophile multifocal minime (6-20 cellules) |
| 2 | Infiltrat inflammatoire neutrophile multifocal modéré (atteinte sous-muqueuse) (21-60 cellules) |
| 3 | Infiltrat inflammatoire neutrophile multifocal sévère (atteinte sous-muqueuse importante pouvant impliquer toute la paroi (61-100 cellules) |
| 4 | Score 3 avec des abcès lésion murale importante (>100 cellules) |

| **3. Lésion épithéliale** | |
|---|---|
| 0 | Absence de lésion épithéliale |
| 1 | Lésion épithéliale multifocale superficielle minime. Desquamation épithéliale |
| 2 | Lésion épithéliale multifocale superficielle modérée. Erosion muqueuse (maintien de l'architecture ou perte de 1-10 cellules). |
| 3 | Lésion épithéliale multifocale superficielle sévère +/- pseudomembranes. Ulcération muqueuse (destruction de la lamina propria ou perte de >10 cellules). |
| 4 | Score 3 avec importantes pseudomembranes ou ulcération épithéliale (perte focale complète de l'épithélium) |

| **4. Perte de cellules caliciformes (cellules à mucus) (400x)** | |
|---|---|
| 0 | >28 |
| 1 | 11-28 |
| 2 | 1-10 |
| 3 | <1 |

### XII. Dosages de cytokines par ELISA

Les niveaux de la cytokine pro-inflammatoire IL-8 dans les surnageants de culture des cellules Caco-2 ont été évalués par ELISA en utilisant le test *Human IL-8 ELISA KIT(4Abio,* Chine) selon les instructions du fabricant. La limite de détection était de 4 pg/mL.

### XIII.Test statistique

Les moyennes ± écart-types des variables étudiées ont été obtenus à partir des trois réplicats ; l'analyse statistique des résultats a été réalisée par le test de Mann-Whitney. Les valeurs de P < 0,01 ou de P < 0,05 sont considérées comme statistiquement significatives.

### Résultats :

### I. Recherche in silico des miRNAs potentiellement impliqués dans la régulation de la réponse inflammatoire induite par C. difficile et sa flagelline FliC

Les résultats de travaux récents montrent que C. *difficile* et sa flagelline FliC induisent une réponse pro-inflammatoire dans les cellules épithéliales intestinales et dans le caecum de souris (modèle murin d'ICD), et que la voie de signalisation NF-κB en est une voie prédominante [16, 17]. Etant donné que les miRNAs sont capables de moduler les voies de signalisation en agissant sur l'ARNm, le rôle de ces molécules a été étudié dans la régulation de la voie de signalisation pro-inflammatoire NF-κB induite par l'interaction de la flagelline FliC avec son récepteur spécifique TLR5 (récepteur de la réponse innée) durant l'ICD.

Une recherche *in silico* a été réalisée à l'aide de 3 algorithmes différents de prédiction de cible pour les miRNAs utilisés par les banques de données miRGator, TargetScan et miRWalk. Ces algorithmes en ligne sont basés sur la recherche d'une similarité entre la région 3' UTR de la séquence d'ARNm d'intérêt et la « seed séquence » des miRNAs présents dans les bases de données spécifiques à chaque banque. Il a également été tenu compte des publications scientifiques indiquant un lien entre les miRNAs et les différents effecteurs de la signalisation du TLR5 à la suite de la stimulation par les flagelles bactériens.

Cent-treize (113) miRNAs ont été sélectionnés d'après les critères choisis à partir de ces recherches in silico et dans la littérature. Parmi ces miRNAs, une vingtaine de miRNA (Tableau 1) ont été testés pour évaluer leur cinétique d'expression dans des cellules Caco-2 mises en contact avec la flagelline FliC de C. *difficile,* plus particulièrement durant les premières heures de contact. Parmi tous ces miRNA testés, le miRNA « miR-27a-5p « a montré une surexpression significative, notamment durant les premières heures d'incubation (Fig. 1A). Sa surexpression a été confirmée lorsque les cellules ont été infectées par la souche R20291 de C. *difficile* à 2 h d'incubation, alors qu'aucune surexpression n'a été observée lorsque les cellules ont été infectées par le mutant non flagellé (Fig. 1B).

### II. Le miR-27a-5p joue un rôle dans la voie de signalisation pro-inflammatoire NF-κB induite par C. difficile et sa flagelline FliC.

Afin d'analyser l'impact de l'inhibition des voies de signalisation NF-κB et MAPKs (ERK1/2, p28 et JNK) sur l'expression du mi-RNA identifié, ces voies ont été inhibées chimiquement par des inhibiteurs spécifiques d'IKKα (voie de NF-κB), et des MAPKs ERK1/2, p38 et JNK, 1 h avant la stimulation des cellules par FliC ou par les bactéries. Après 2 h d'incubation des cellules avec FliC ou la souche R20291, l'expression du miRNA-27a-5p a été abolie (Fig. 2A et B). Une inhibition beaucoup plus forte (d'environ 40 fois) de l'expression du miR-27a-5p a été obtenue en utilisant l'inhibiteur d'IKKα (BMS 345541) démontrant que ce miRNA joue un rôle dans la régulation de la voie de signalisation pro-inflammatoire NF-κB induite par C. *difficile* et sa flagelline (Fig. 2A et B).

### III. Le miR-27a-5p-mimic inhibe la voie de signalisation pro-inflammatoire NF-κB induite par C. difficile et sa flagelline FliC in vitro.

Les résultats précédant démontrent que le miR-27a-5p (SEQ ID NO :1 = AGGGCUUAGCUGCUUGUGAGCA), dont l'expression est induite par C. *difficile* et sa flagelline FliC via l'activation de NF-κB, joue un rôle dans la régulation de cette voie NF-κB. Le rôle de miR-27a-5p a été caractérisé pour révéler s'il s'agit d'un rôle activateur (pro-inflammatoire) ou inhibiteur (anti-inflammatoire) de la voie NF-κB. Pour ce faire, différentes molécules ont été utilisées : une molécule (oligonucléotidique) mimant l'activité de ce mR-27a-5p (« mR-27a mimic ») ou une molécule chimique antagonisant son activité (« miR-27a-inhibitor » = Anti-hsa-miR-27a-5p).

Après transfection des cellules Caco-2 par ces deux molécules, ainsi que par les oligonucléotides et inhibiteurs contrôles, l'expression du miR-27a-5p a été mesurée après lyse cellulaire. Comme indiqué dans la Fig. 3A, l'inhibiteur du miR-27a-5p réduit significativement l'expression de ce miRNA dans les cellules stimulées par FliC, alors que le miR-27a mimic est fortement exprimé. L'effet de la transfection des cellules par ces deux molécules a été mesuré quant à la dégradation de l'effecteur IκB-α signant l'activation de la voie NF-κB, l'expression des ARNm des cytokines pro-inflammatoires TNFα et IL-8, ainsi que la production d'IL-8 dans le surnageant de culture cellulaire.

L'anti-miR-27a-5p (miR-27a inhibitor) induit une forte dégradation d'IκB-α, une expression accrue des ARNm des cytokines TNFα et IL-8, ainsi qu'une augmentation de la synthèse d'IL-8 dans le surnageant de culture (Fig. 3B, C et D), suggérant que le miR-27a-5p joue un rôle inhibiteur de la voie NF-κB et donc un rôle anti-inflammatoire dans la cascade de signalisation induite par l'interaction FliC-TLR5.

Tout à fait dans le sens de ces observations, la transfection des cellules par le miR-27a mimic a induit une réponse cellulaire anti-inflammatoire caractérisée par l'absence de dégradation d'IκB-α et donc de l'activation de NF-κB, une forte inhibition de l'expression des ARNm des cytokines TNFα et IL-8, et une absence de synthèse d'IL-8 dans le surnageant cellulaire (Fig. 3B, C et D). L'ensemble de ces observations montre que le miR-27a-5p joue un rôle inhibiteur de la voie pro-inflammatoire NF-κB à la suite de l'activation de cette voie par l'interaction flagelline-TLR5. Ce miRNA interviendrait dans une boucle de rétrocontrôle de la cascade de signalisation TLR5-NF-κB.

### IV. Le miR-27a-5p est fortement surexprimé in vivo dans le caecum des souris infectées par C. difficile.

Afin de confirmer les observations réalisées *in vitro* quant au rôle potentiel du miR-27a-5p dans la régulation de l'inflammation induite par C. *difficile* et son flagelle, l'expression de ce miR-27a-5p a été analysée dans l'intestin (caecum) lors de l'infection sur le modèle murin d'ICD.

Deux groupes de souris femelles âgées de 4 à 5 semaines C57BL/6 à microbiote conventionnel (n = 10 pour chaque groupe) et un groupe de souris C57BL/6 KO *tlr5*^{*-*/*-*}(n'exprimant pas le TLR5, n = 10) ont été constitués. Après le traitement antibiotique des animaux les 6 jours précédant l'infection afin de créer une dysbiose (Cf. Matériel et Méthodes), un groupe de souris à microbiote conventionnel a été infecté par gavage intragastrique avec la souche sauvage R20291 de *C. difficile* (WT) et un autre par le mutant isogénique dépourvu de flagelles (FliC-). Un groupe de souris KO *tlr5*^{*-*/*-*} a également été infecté par la souche WT. Un groupe de souris non infectées a servi comme contrôle pour établir les rapports d'expression relative pour chaque groupe. L'analyse de l'expression du miR-27a-5p dans le caecum des animaux a montré une plus forte expression de ce miRNA dans le caecum des souris C57BL/6 à microbiote conventionnel infectées par la souche WT, par rapport à l'expression observée chez les souris infectées par le mutant non flagellé (FliC-) et celle des souris KO *tlr5*^{*-*/*-*} infectées par la souche WT (Fig. 4A). Ces résultats indiquent que le flagelle de C. *difficile* par son interaction avec le TLR5 joue un rôle dans l'expression accrue du miR-27-a-5p dans le caecum des souris, suggérant qu'in vivo, l'activation de NF-κB à la suite de l'interaction flagelle-TLR5 préalablement reportée [17], est corrélée à l'expression du miR-27a-5p.

Par ailleurs, l'analyse de l'expression de l'ARNm du gène de la cytokine KC chez la souris, équivalent de le I'IL-8 humaine, révèle une parfaite corrélation avec l'expression accrue du miR-27a-5p comme l'indique la Fig. 4B.

### V. Un mimique du miR-27a-5p réduit la sévérité de l'inflammation in vivo dans le modèle muris d'ICD.

Compte tenu des effets anti-inflammatoires induits par le miR-27a-5p dans les cellules Caco-2, l'effet in vivo d'un oligonucléotide mimique de ce miRNA (miR-27a-5p-mimic) a été analysé. Cet oligonucléotide a été administré par voie intraveineuse (IV) dans le modèle murin d'ICD (Fig. 5). Trois groupes de souris femelles âgées de 4 à 5 semaines C57BL/6 à microbiote conventionnel (n = 9-10 pour chaque groupe) ont été testés. Après le traitement antibiotique induisant la dysbiose, un groupe d'animaux non infectés a été traité par la suspension miR-27a-5p mimic / in vivo-jetPEl^{®} à raison de 3 doses de 50 µg (soit 25 mg/kg), au moment de l'infection, 8 h et 24 h après l'infection, par voie intra-veineuse (150 µL injectés en rétro-orbital). Un deuxième groupe d'animaux a été infecté par la souche sauvage R20291 et non traité par le miR-mimic et un troisième groupe a été également infecté par cette souche de C. *difficile* mais traité par la suspension miR-27a-5p mimic / in vivo-jetPEl^{®} comme pour le premier groupe.

À la suite de l'infection, comme attendu dans ce modèle d'ICD, les souris infectées et non traitées par le miR-27a-mimic, ont développé de signes cliniques d'ICD : diarrhée, poil hérissé et diminution de l'activité à J1 et J2 post-infection (Tableau 4). Seuls 3 animaux sur 10 du groupe infecté et traité par le miR27-a-mimic ont développé une diarrhée, et ce à J2 post-infection. Il est à noter que le taux de colonisation intestinal des animaux infectés quantifié par le dénombrement bactérien dans le contenu caecal (unités formatrices de colonies - UFC/g) à J2 post-infection est tout à fait équivalent entre les groupes de souris infectées (WT non traité et WT + miR-27a-mimic) excluant la possibilité que la différence des manifestations cliniques entre ces deux groupes soit le résultat d'une faible colonisation intestinale des souris.

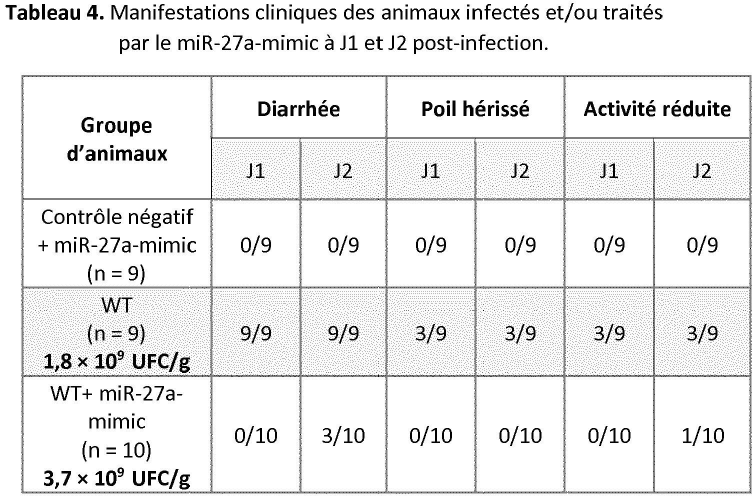

A J2 post-infection, l'analyse anatomo-pathologique du caecum des animaux infectés par la souche sauvage et non traités, a montré un aspect hyperémique inflammatoire avec des zones hémorragiques et un contenu caecal muqueux comparé au caecum normal des souris non infectées (contrôle négatif) comme montré dans la Fig. 6A et C. L'aspect inflammatoire du caecum a été largement atténué dans le groupe des souris infectées et traités par le miR-27a-mimic (Fig. 6E). En ce qui concerne l'étude histologique des coupes de caecums, comme attendu, un œdème important de la muqueuse, avec un infiltrat inflammatoire, une érosion épithéliale et une perte de cellules de gobelet sont retrouvés dans le caecum des souris infectées par la souche sauvage et non traitées par le miR-27a-mimic, avec un score d'inflammation (Tableau 3) allant de 6 à 9, comparé à un score 0 pour les souris non infectées (Fig. 6G). En revanche, dans le groupe d'animaux infectés et traités par le miR-27-a-mimic, 6 souris sur 10 ont présenté un caecum indemne ou avec peu de signes d'inflammation et un contenu caecal normal et sans mucus (Fig. 6E). Le score histologique d'inflammation était inférieur à 6 pour ces animaux qui présentaient principalement un infiltrat cellulaire et un œdème léger à modéré (Fig. 6F et G). Les 3 souris de ce groupe « infectées et traités par le mimic » ayant présenté une diarrhée à J2 post-infection (Tableau 4) ont obtenu un score d'inflammation élevé et comparable aux souris infectées et non traités (Fig 6G), ce qui ramène le taux d'efficacité du miR-27a-5p à 70 % sur ce modèle murin d'infection.

L'analyse de l'activation de la voie NF-κB dans le tissu caecal à J2 post-infection, a révélé une forte dégradation d'IκB-α dans le caecum des souris infectées et non traitées, comparé aux souris non infectées, ce qui correspond, comme pour les manifestations cliniques, aux observations habituelles sur ce modèle d'ICD [17]. En revanche, aucune activation d'IκB-α n'a été détectée dans le caecum des souris infectées et traitées par le miR-27a-mimic (Fig. 6H) ce qui renforce les observations précédentes quant à la réduction des manifestations cliniques et des phénomènes inflammatoires intestinaux associés à l'infection par la bactérie sur ce modèle murin.

### CONCLUSION

En conclusion, les présents travaux mettent en lumière le rôle capital d'un miRNA, le miR-27a-5p pour diminuer la réponse inflammatoire induite par les toxines et les flagelles des bactéries C. *difficile* et ainsi prévenir ou diminuer les lésions muqueuses causées par cette inflammation. En inhibant la voie de signalisation NF-κB activée par l'interaction flagelles-TLR5 au cours de l'infection intestinale par C. *difficile,* ce miRNA impacte la présentation clinique ainsi que le pronostic de l'ICD.

L'effet *in vivo* d'un miR-27a-5p-mimic spécifique ciblant la voie de signalisation pro-inflammatoire TLR5-NF-κB induite par C. *difficile* a été démontré dans un modèle murin d'ICD. Cette molécule est capable de réduire la réponse délétère des souris infectées par C. *difficile,* en diminuant la réponse inflammatoire intestinale chez ces souris. Ces résultats prouvent qu'il est possible de diminuer les effets délétères sévères dus à l'infection par cette bactérie, en utilisant ce miRNA en tant que principe actif dans une composition thérapeutique.

### Références bibliographiques

1. Cloud, J. and C.P. Kelly, Update on Clostridium difficile associated disease. Curr Opin Gastroenterol, 2007. 23(1): p. 4-9.
2. Heinlen, L. and J.D. Ballard, Clostridium difficile infection. Am J Med Sci, 2010. 340(3): p. 247-52.
3. Dubberke, E.R. and M.A. Olsen, Burden of Clostridium difficile on the healthcare system. Clin Infect Dis, 2012. 55 Suppl 2: p. S88-92.
4. Miller, M.A., et al., Morbidity, mortality, and healthcare burden of nosocomial Clostridium difficile-associated diarrhea in Canadian hospitals. Infect Control Hosp Epidemiol, 2002. 23(3): p. 137-40.
5. Carrico, R.M., Silent menace. C. difficile and its threat to health care facilities. Health Facil Manage, 2011. 24(6): p. 43-5.
6. Dubberke, E.R. and A.I. Wertheimer, Review of current literature on the economic burden of Clostridium difficile infection. Infect Control Hosp Epidemiol, 2009. 30(1): p. 57-66.
7. O'Brien, J.A., et al., The emerging infectious challenge of clostridium difficile-associated disease in Massachusetts hospitals: clinical and economic conséquences. Infect Control Hosp Epidemiol, 2007. 28(11): p. 1219-27.
8. Rebmann, T. and R.M. Carrico, Preventing Clostridium difficile infections: an executive summary of the Association for Professionals in Infection Control and Epidemiology's élimination guide. Am J Infect Control, 2011. 39(3): p. 239-42.
9. McGlone, S.M., et al., The economic burden of Clostridium difficile. Clin Microbiol Infect, 2012. 18(3): p. 282-9.
10. Kuijper, E.J., B. Coignard, and P. Tull, Emergence of Clostridium difficile-associated disease in North America and Europe. Clin Microbiol Infect, 2006. 12 Suppl 6: p. 2-18.
11. Wiegand, P.N., et al., Clinical and economic burden of Clostridium difficile infection in Europe: a systematic review of healthcare-facility-acquired infection. J Hosp Infect, 2012. 81(1): p. 1-14.
12. Eckert, C. and F. Barbut, [Clostridium-difficile-associated infections]. Med Sci (Paris), 2010. 26(2): p. 153-8.
13. Kuehne, S.A., et al., The role of toxin A and toxin B in Clostridium difficile infection. Nature, 2010. 467(7316): p. 711-3.
14. Lyras, D., et al., Toxin B is essential ƒor virulence of Clostridium difficile. Nature, 2009. 458(7242): p. 1176-9.
15. Tasteyre, A., et al., Role of FliC and FliD ƒlagellar proteins of Clostridium difficile in adherence and gut colonization. Infect Immun, 2001. 69(12): p. 7937-40.
16. Batah, J., et al., Clostridium difficile ƒlagella predominantly activate TLR5-linked NF-kappaB pathway in epithelial cells. Anaerobe, 2016. 38: p. 116-24.
17. Batah, J., et al., Clostridium difficile ƒlagella induce α pro-inflammatory response in intestinal epithelium oƒ mice in cooperation with toxins. Sci Rep, 2017. 7(1): p. 3256.
18. Bentwich, I., et al., Identification oƒ hundreds oƒ conserved and nonconserved human microRNAs. Nat Genet, 2005. 37(7): p. 766-70.
19. He, X., Z. Jing, and G. Cheng, MicroRNAs: new regulators oƒ Toll-like receptor signalling pathways. Biomed Res Int, 2014. 2014: p. 945169.
20. Taganov, K.D., et al., NF-kappaB-dependent induction of microRNA miR-146, an inhibitor targeted to signaling proteins oƒ innate immune responses. Proc Natl Acad Sci USA, 2006. 103(33): p. 12481-6.
21. Bader, A.G., et al., Developing therapeutic microRNAs ƒor cancer. Gene Ther, 2011. 18(12): p. 1121-6.
22. Minton, N., et al., The development oƒ Clostridium difficile genetic systems. Anaerobe, 2004. 10(2): p. 75-84.
23. Häsler R, et al. 2012. Microbial pattern recognition causes distinct functional micro-RNA signatures in primary human monocytes. PloS One 7:e31151.
24. Norman E Buroker X-HN. 2013. Medical Diagnostic Methods Circulating miRNAs from Dried Blood Spots are Associated with High Altitude Sickness.
25. Romay MC, et al. 2015. Regulation of NF-κB signaling by oxidized glycerophospholipid and IL-1β induced miRs-21-3p and -27a-5p in human aortic endothelial cells. J Lipid Res 56:38-50.
26. Nahid MA, et al. 2013. Regulation of TLR2-mediated tolerance and cross-tolerance through IRAK4 modulation by miR-132 and miR-212. J Immunol Baltim Md 1950 190:1250-1263.
27. Liu G, et al. 2009. miR-147, a microRNA that is induced upon Toll-like receptor stimulation, regulates murine macrophage inflammatory responses. Proc Natl Acad Sci U S A 106:15819-15824.
28. Ceppi M, et al. 2009. MicroRNA-155 modulates the interleukin-1 signaling pathway in activated human monocyte-derived dendritic cells. Proc Natl Acad Sci U S A 106:2735-2740.
29. Lu Z, et al. 2011. miR-301a as an NF-kB activator in pancreatic cancer cells. EMBO J 30:57-67.
30. Wang B, et al. 2011. Systematic Evaluation of Three microRNA Profiling Platforms: Microarray, Beads Array, and Quantitative Real-Time PCR Array. PLoS ONE 6:e17167.
31. Häsler R, et al. 2012. Microbial pattern recognition causes distinct functional micro-RNA signatures in primary human monocytes. PloS One 7:e31151.
32. Norman E Buroker X-HN. 2013. Medical Diagnostic Methods Circulating miRNAs from Dried Blood Spots are Associated with High Altitude Sickness.
33. Nahid MA, et al. 2013. Regulation of TLR2-mediated tolerance and cross-tolerance through IRAK4 modulation by miR-132 and miR-212. J Immunol Baltim Md 1950 190:1250-1263.
34. Liu G, et al. 2009. miR-147, a microRNA that is induced upon Toll-like receptor stimulation, regulates murine macrophage inflammatory responses. Proc Natl Acad Sci U S A 106:15819-15824.
35. Ceppi M, et al. 2009. MicroRNA-155 modulates the interleukin-1 signaling pathway in activated human monocyte-derived dendritic cells. Proc Natl Acad Sci U S A 106:2735-2740.
36. Lu Z, et al. 2011. miR-301a as an NF-kB activator in pancreatic cancer cells. EMBO J 30:57-67.
37. Wang B, et al. 2011. Systematic Evaluation of Three microRNA Profiling Platforms: Microarray, Beads Array, and Quantitative Real-Time PCR Array. PLoS ONE 6:e17167.
38. Martimprey H et al, 2010. New Core-Shell nanoparticules for the intravenous delivery of siRNA to experimental thyroid papillary carcinoma, Pharmaceutical Research, 2010.
39. Reigadas Ramírez E, Bouza ES. Economic Burden of Clostridium difficile Infection in European Countries. Adv Exp Med Biol. 2018;1050:1-12
40. Rupaimoole R. & Slack F.J., MicroRNA therapeutics: towards a new era for the management of cancer and other diseases. Nat Rev Drug Discov. 2017 Mar;16(3):203-222.
41. Lagos-Quintana M, Rauhut R, Lendeckel W, Tuschl T. Identification of novel genes coding for small expressed RNAs. Science. 2001 Oct 26;294(5543):853-8.
42. Romay et al Regulation of NF-kB signaling by oxidized glycerophospholipid and IL-1β induced miRs-21-3p and -27a-5p in human aortic endothelial cells. J Lipid Res. 2015 Jan;56(1):38-50
43. Wu et al Coordinated Targeting of the EGFR Signaling Axis by MicroRNA- 27a∗ Oncotarget. 2013 Sep;4(9):1388-98
44. Geary RS, Pharmacokinetic properties of 2'-O-(2-methoxyethyl)-modified oligonucleotide analogs in rats. J Pharmacol Exp Ther. 2001 Mar;296(3):890-7.
45. Baumann V. and Winckler J. miRNA-based therapies: strategies and delivery platforms for oligonucleotide and non-oligonucleotide agents. Future Med Chem. 2014;6(17):1967-84
46. Chen H. miR-27a protects human mitral valve interstitial cell from TNF-α-induced inflammatory injury via up-regulation of NELL-1. Braz J Med Biol Res. 2018; 51(6)
47. Arros-Silva D, Costa-Pinheiro P, Duarte H, Sousa EJ, Evangelista AF, Graça I, et al. MicroRNA-27a-5p regulation by promoter methylation and MYC signaling in prostate carcinogenesis. Cell Death Dis. 7 févr 2018;9(2):167
48. Mertens-Talcott SU, Chintharlapalli S, Li X, Safe S. The oncogenic microRNA-27a targets genes that regulate specificity protein transcription factors and the G2-M checkpoint in MDA-MB-231 breast cancer cells. Cancer Res. 15 nov 2007;67(22):11001-11.
49. Xie N. et al, miR-27a regulates inflammatory response of macrophages by targeting IL-10. J Immunol. 2014 Jul 1; 193(1): 327-334.
50. Pfeiffer D. et al. miR-146a, miR-146b, and miR-155 increase expression of IL-6 and IL-8 and support HSP10 in an In vitro sepsis model. PLOS ONE June 29, 2017
51. Gao et al, MicroRNA-146 regulates the inflammatory cytokines expression in vascular endothelial cells during sepsis. Die Pharmazie - An International Journal of Pharmaceutical Sciences, Volume 72, Number 11, November 2017, pp. 700-704(5)

## Revendications

1. Composition pharmaceutique contenant un ARN double-brin mimique du miR-27a-5p pour son utilisation pour prévenir ou diminuer les lésions intestinales chez un sujet souffrant d'une infection à *Clostridium Difficile.*

2. Composition pharmaceutique pour son utilisation selon la revendication 1, dans laquelle ledit ARN mimique du miR-27a-5p a une séquence nucléotidique similaire à plus de 70% à SEQ ID NO :1.

3. Composition pharmaceutique pour son utilisation selon la revendication 1, dans laquelle ledit ARN mimique du miR-27a-5p a la séquence nucléotidique SEQ ID NO :1.

4. Composition pharmaceutique pour son utilisation selon l'une des revendications 1 à 3, comprenant en outre un excipient pharmaceutiquement acceptable.

5. Composition pharmaceutique pour son utilisation selon l'une des revendications 1 à 4, dans laquelle ledit ARN mimique du miR-27a-5p est associé à ou inclus dans un vecteur, une matrice ou des particules favorisant sa stabilité et/ou sa transfection.

6. Composition pharmaceutique pour son utilisation selon la revendication 5, dans lequel ledit vecteur est choisi parmi : les adénovirus, les rétrovirus, les lentivirus, les virus adéno-associé (AAV), les virus de l'herpès, les cytomégalovirus (CMV), et les virus de la vaccine.

7. Composition pharmaceutique pour son utilisation selon la revendication 5, dans lequel lesdites particules sont choisies parmi : les liposomes (DOPC), les nanoparticules lipidiques, les nanocellules, les nanoparticules de silice, les exosomes.

8. Composition pharmaceutique pour son utilisation selon la revendication 5, dans lequel ladite matrice est choisie parmi l'émulsion lipidique neutre (NLE), le polyethylenimine (PEI), le poly(lactide-co-glycolide) (PLGA).

9. Composition pharmaceutique pour son utilisation selon l'une des revendications 1 à 4, dans lequel ledit ARN mimique du miR-27a-5p est conjugué à une molécule chimique choisie parmi : les lipides, le cholesterol, le PEG, la cyclodextrine, le chitosan, les dendrimères (de poly(amidoamine) ou de poly(propylenimine), le N-acetyl-D-galactosamine (GalNAc).

10. Composition pharmaceutique pour son utilisation selon l'une des revendications 1 à 9, **caractérisée en ce qu'**elle est administrée chez ledit sujet par voie intraveineuse.

## Patentansprüche

1. Pharmazeutische Zusammensetzung, enthaltend eine doppelsträngige RNA, die miR-27a-5p nachahmt, zu deren Verwendung zur Verhinderung oder Verringerung von Darmläsionen bei einem Probanden, der an einer *Clostridium-difficile-Infektion* leidet.

2. Pharmazeutische Zusammensetzung zu deren Verwendung nach Anspruch 1, wobei die RNA, die miR-27a-5p nachahmt, eine Nukleotidsequenz aufweist, die zu mehr als 70% der SEQ ID Nr. 1 ähnlich ist.

3. Pharmazeutische Zusammensetzung zu deren Verwendung nach Anspruch 1, wobei die RNA, die miR-27a-5p nachahmt, die Nukleotidsequenz SEQ ID Nr. 1 aufweist.

4. Pharmazeutische Zusammensetzung zu deren Verwendung nach einem der Ansprüche 1 bis 3, die ferner einen pharmazeutisch unbedenklichen Hilfsstoff umfasst.

5. Pharmazeutische Zusammensetzung zu deren Verwendung nach einem der Ansprüche 1 bis 4, wobei die RNA, die miR-27a-5p nachahmt, mit einem Vektor, einer Matrix oder Partikeln, die ihre Stabilität und/oder ihre Transfektion begünstigen, assoziiert ist oder in einen Vektor, eine Matrix oder Partikel, die ihre Stabilität und/oder ihre Transfektion begünstigen, eingebunden ist.

6. Pharmazeutische Zusammensetzung zu deren Verwendung nach Anspruch 5, wobei der Vektor ausgewählt ist aus: Adenoviren, Retroviren, Lentiviren, adenoassoziierten Viren (AAV), Herpesviren, Zytomegalieviren (ZMV) und Vakziniaviren.

7. Pharmazeutische Zusammensetzung zu deren Verwendung nach Anspruch 5, wobei die Partikel ausgewählt sind aus: Liposomen (DOPC), Lipid-Nanopartikeln, Nanozellen, Siliciumdioxid-Nanopartikeln, Exosomen.

8. Pharmazeutische Zusammensetzung zu deren Verwendung nach Anspruch 5, wobei die Matrix aus neutraler Lipidemulsion (NLE), Polyethylenimin (PEI), Poly(lactid-co-glycolid) (PLGA) ausgewählt ist.

9. Pharmazeutische Zusammensetzung zu deren Verwendung nach einem der Ansprüche 1 bis 4, wobei die RNA, die miR-27a-5p nachahmt, an ein chemisches Molekül konjugiert ist, das ausgewählt ist aus: Lipiden, Cholesterin, PEG, Cyclodextrin, Chitosan, Dendrimeren (von Poly(amidoamin) oder Poly(propylenimin)), N-Acetyl-D-galactosamin (GalNAc).

10. Pharmazeutische Zusammensetzung zu deren Verwendung nach einem der Ansprüche 1 bis 9, **dadurch gekennzeichnet, dass** sie intravenös an den Probanden verabreicht wird.

## Claims

1. Pharmaceutical composition containing a double strand RNA mimic of miR-27a-5p, for use thereof for preventing or reducing intestinal lesions in a subject suffering from an infection with *Clostridium Difficile.*

2. Pharmaceutical composition for use thereof according to claim 1, wherein said RNA mimic of miR-27a-5p has a nucleotide sequence more than 70% similar to SEQ ID NO: 1.

3. Pharmaceutical composition for use thereof according to claim 1, wherein said RNA mimic of miR-27a-5p has the nucleotide sequence SEQ ID NO: 1.

4. Pharmaceutical composition for use thereof according to one of claims 1 to 3, further comprising a pharmaceutically acceptable excipient.

5. Pharmaceutical composition for use thereof according to one of claims 1 to 4, wherein said RNA mimic of miR-27a-5p is associated with or included in a vector, a matrix or particles promoting its stability and/or its transfection.

6. Pharmaceutical composition for use thereof according to claim 5, wherein said vector is chosen from: adenoviruses, retroviruses, lentiviruses, adeno-associated viruses (AAV), herpes viruses, cytomegaloviruses (CMV) and viruses of the vaccine.

7. Pharmaceutical composition for use thereof according to claim 5, wherein said particles are chosen from: liposomes (DOPC), lipid nanoparticles, nanocells, nanoparticles of silica, exosomes.

8. Pharmaceutical composition for use thereof according to claim 5, wherein said matrix is chosen from neutral lipid emulsion (NLE), polyethylenimine (PEI), poly(lactic-co-glycolic acid) (PLGA).

9. Pharmaceutical composition for use thereof according to one of claims 1 to 4, wherein said RNA mimic of miR-27a-5p is conjugated with a chemical molecule chosen from: lipids, cholesterol, PEG, cyclodextrin, chitosan, dendrimers (of poly(amidoamine) or poly(propylenimine), N-acetyl-D-galactosamine (GalNAc).

10. Pharmaceutical composition for use thereof according to one of claims 1 to 9, **characterised in that** it is administered to the subject intravenously.
